# EUROPEAN PATENT SPECIFICATION

(11) **EP 3 494 145 B1**
(45) Date of publication and mention of the grant of the patent: **20.05.2020**
(21) Application number: 17745751.2
(22) Date of filing: 02.08.2017
(51) Int. Cl.: C08B 37/08, A61K 31/728

(54) **METHOD OF CROSSLINKING GLYCOSAMINOGLYCANS**
VERFAHREN ZUR VERNETZUNG VON GLYKOSAMINOGLYKANEN
PROCÉDÉ DE RÉTICULATION DE GLYCOSAMINOGLYCANES

(30) Priority: 03.08.2016 US 201662370479 P; 23.12.2016 EP 16206622
(43) Date of publication of application: 12.06.2019
(73) Proprietor: Galderma Research & Development, 06410 Biot (FR); Centre National de la Recherche Scientifique, 75794 Paris (FR)
(72) Inventor: AUZELY-VELTY, Rachel, 38450 Le Gua (FR); BOITEAU, Jean-Guy, 06650 Opio (FR); FIGUEIREDO, Tamiris, 38400 Saint Martin d'Hères (FR); GERFAUD, Thibaut, 06370 Mouans Sartoux (FR); HARRIS, Craig Steven, 06410 Biot (FR); JING JING, Laura, 06600 Antibes (FR); TOMAS, Loïc, 06410 Le Biot (FR)
(74) Representative: Lavoix
(86) International application number: PCT/EP2017/069576
(87) International publication number: WO 2018/024795

(56) References cited:
- WO-A1-2014/072330
- US-A1- 2013 129 797
- US-A1- 2014 155 305

## Description

### Technical field of the invention

The present invention relates to the field of hydrogels containing crosslinked polysaccharides and the use of such hydrogels in medical and/or cosmetic applications. More specifically, the present invention is concerned with hydrogels made of crosslinked glycosaminoglycans, particularly crosslinked hyaluronic acid, chondroitin or chondroitin sulfate, having reversible linkages, preferably boronate ester bonds, leading to new benefits.

### Background of the invention

Water-absorbing gels, or hydrogels, are widely used in the biomedical field. They are generally prepared by chemical crosslinking of polymers to infinite networks. While many polysaccharides absorb water until they are completely dissolved, crosslinked gels of the same polysaccharides can typically absorb a certain amount of water until they are saturated, i.e. they have a finite liquid retention capacity, or swelling degree.

Hyaluronic acid, chondroitin and chondroitin sulfate are well-known biocompatible polymers. They are naturally occurring polysaccharides belonging to the group of glycosaminoglycans (GAGs). All glycosaminoglycans are negatively charged heteropolysaccharide chains which have a capacity to absorb large amounts of water.

Chondroitin sulfate (CS) is a highly abundant glycosaminoglycan found in the connective tissues of mammals where it, together with other sulfated glycosaminoglycans, is bound to proteins as part proteoglycans. It has previously been shown that hydrogels containing CS successfully can be used in biomedical applications due to their resemblance to the natural extra cellular matrix (Lauder, R.M., Complement Ther Med 17: 56-62, 2009). Chondroitin sulfate is also used in the treatment of osteoarthritis, e.g. as a dietary supplement.

Crosslinking of the glycosaminoglycans prolongs the duration of the degradable polymers that make up the network, which is useful in many applications.

However, one of the main drawbacks of a large majority of the glycosaminoglycans-based gels, such as when used for treating wrinkles lies in the difficulty of injecting the hydrogel due to the high crosslinking density of the polysaccharide.

Hyaluronic acid is one of the most widely used biocompatible polymers for medical use. Hyaluronic acid and the other glycosaminoglycans are negatively charged heteropolysaccharide chains which have a capacity to absorb large amounts of water. Hyaluronic acid and products derived from hyaluronic acid are widely used in the biomedical and cosmetic fields, for instance during viscosurgery and as a dermal filler.

Since hyaluronic acid is present with identical chemical structure except for its molecular mass in most living organisms, it gives a minimum of foreign body reactions and allows for advanced medical uses. Crosslinking and/or other modifications of the hyaluronic acid molecule is typically necessary to improve its duration *in vivo.* Furthermore, such modifications affect the liquid retention capacity of the hyaluronic acid molecule. As a consequence thereof, hyaluronic acid has been the subject of many modification attempts.

In the prior art, the hydrogels are prepared by reacting hyaluronic acid, for example, with BDDE (butanediol diglycidyl ether) in a basic aqueous medium resulting in the formation of covalent linkages (WO 97/04012). This is not a reversible process. WO 2014/072330 discloses a polymer composition comprising a mixture of phenylboronic acid modified hyaluronic acid polymer grafted on at least a hydroxyl with a group comprising phenylboronic acid and a cis-diol modified HA polymer grafted on at least a hydroxyl with a group comprising a cis-diol. US 2014/0155305 discloses an aqueous solution comprising a thickening polymer with diol groups distributed along it, such as guar or other polysaccharide, which is cross linked with a cross-linker which contains a plurality of boroxole groups. US 2013/0129797 A1 discloses polymeric compositions that comprise at least one polymer residue and at least one crosslinking moiety, wherein the polymer residue is crosslinked by the crosslinking moiety and wherein the crosslinking moiety is formed from a reaction between a boronic acid moiety and a hydroxamic acid moiety.

### Description of the invention

It is an object of the present invention to provide a hydrogel having a glycosaminoglycan (GAG) as the swellable polymer, having reversible linkages.

It is also an object of the present invention to provide a method for preparing hydrogels of glycosaminoglycan molecules by mild and efficient routes. It is also an object of the invention to provide a simpler method for manufacturing crosslinked glycosaminoglycans, preferably with gel properties.

One object of the invention is to provide crosslinked glycosaminoglycans with less chemical modifications and/or a simpler structure.

Yet another object of the invention is to mitigate, alleviate or eliminate one or more of the drawbacks of the prior art.

The present invention concerns new hydrogel which show the following benefits:
- Easier to inject,
- More malleable,
- can self-repair.

The invention also concerns the use of such gels, of particular interest to fill wrinkles and / or shape the face more accurately and with fewer traumas for the patient.

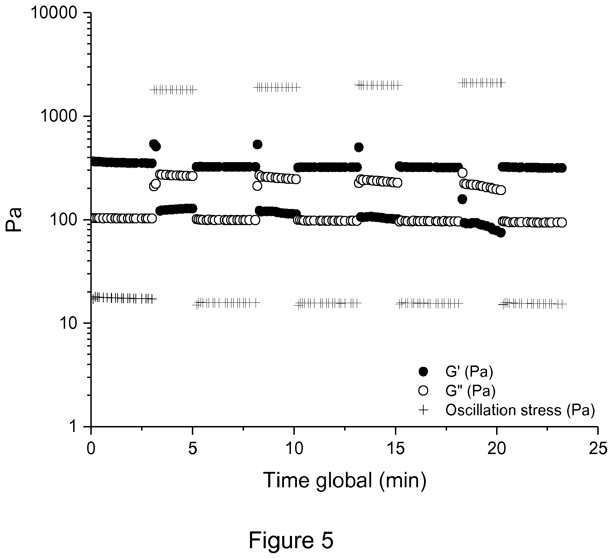

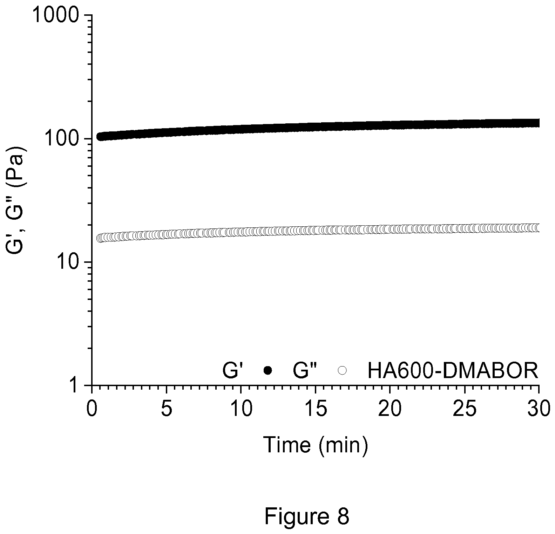

In one aspect of the invention, there is provided, a method of cross-linking a first glycosaminoglycan having a backbone diol function and a second glycosaminoglycan being grafted with a boronate hemiester, comprising crosslinking said first glycosaminoglycan with said second glycosaminoglycan by forming an alkoxyboronate ester anion linkage between the boronate hemiester of the second glycosaminoglycan with the backbone diol function of said first glycosaminoglycan. In other words, a method of cross-linking glycosaminoglycans, comprising crosslinking a first glycosaminoglycan with a second glycosaminoglycan, said second glycosaminoglycan being grafted with a boronate hemiester capable of forming a alkoxyboronate ester anion with a backbone diol function of said first glycosaminoglycan. The method does not exclude additional crosslinking. The method according to the invention uses the high affinity towards diols of a boronate hemiester function to bind directly to a diol function of the backbone of a glycosaminoglycan and to form a gel without the need for binding via a sugar moiety grafted onto a second glycosaminoglycan. In WO 2014/072330, phenylboronic acid is used to crosslink hyaluronic acid, which does not allow for direct binding directly to a backbone diol function of said first glycosaminoglycan (comparison in Example 3). The method according to the invention is thus a simpler method of crosslinking glycosaminoglycans, requiring less synthetic steps, less modification and resulting in a less complex structure. As demonstrated in the appended examples a gel is formed by using a boronate hemiester grafted to a glycosaminoglycan without the need for grafting sugar moieties or other moieties to a second glycosaminoglycan (example 1, 2 and 3). A glycosaminoglycan grafted with a boronate hemiester further provides self-healing properties to the obtained gel (see Figure 5, Example 3; Figure 8, Example 15). The gel produced by the method according to the invention is also easy to inject as the reversible bonds break when pushed through the syringe, and then quickly reform inside the body. The gels can be injected as preformed solids, because the solid gel can manage external damages and repair itself under a proper shear stress. Due to fast gelation kinetics after extrusion/injection, they recover their solid form almost immediately. Thus, before the gel reforms inside the body, the gel is malleable, until the reversible bonds reform. Thus, in one embodiment, the method provides a self-healing gel.

A glycosaminoglycan can be grafted to a higher degree of substitution with a boronate hemiester than a corresponding glycosaminoglycan grafted with a phenylboronic acid.

As used herein, the term "backbone" refers to the polysaccharide chain in its native form i.e. groups grafted to the backbone are not part of the backbone. As an example, below the backbone of hyaluronic acid is shown.

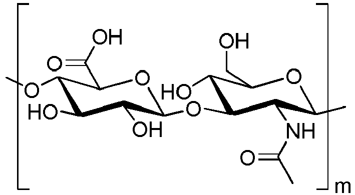

As used herein, the term "boronate hemiester" is to be interpreted as a compound of general formula BR(OR)(OH), as opposed to a boronic acid, which has a general formula BR(OH)₂, or a boronate ester which has a general formula BR(OR)₂. Each R, in this context, may independently represent any organic moiety since the purpose of these formulae relates to different boron functional groups.

A boronate ester is in equilibrium with its tetrahedral anionic form in water (below). The anionic form is an hydroxyboronate ester anion (Hall, D.G., 2011, Boronic Acids: Preparation and Applications in Organic Synthesis, Medicine and Materials, Second Edition, Wiley-VCH Verlag GmbH & Co.).

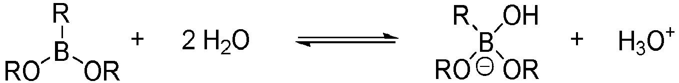

Thus, in general terms, an "alkoxyboronate ester anion" is to be understood as an anionic tetrahedral form, formed between a boronate ester and any alkoxy group, substituted or unsubstituted. An "alkoxyboronate ester anion" according to the invention, is an "alkoxyboronate ester anion" formed between a boronate hemiester and a backbone diol function of a glycosaminoglycan (below).

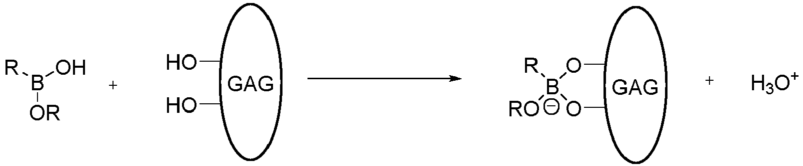

In one embodiment of this aspect of the invention the boronate hemiester is a compound comprising a 5-6-membered cyclic boronate hemiester moiety, sometimes referred to as a boroxole (Kotsubayashi et al. ACS Macro Lett. 2013, 2, 260-264). A five-membered boroxole is referred to as an oxaborole and a six-membered, an oxaborinine, see below. Thus, in one embodiment of this aspect of the invention the boronate hemiester is a compound comprising an oxaborole or an oxaborinine moiety. A glycosaminoglycan grafted with a boroxole is shown in the appended examples to give rise to a gel upon cross-linking.

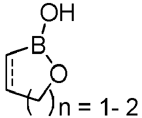

In one embodiment of this aspect of the invention the boronate hemiester is an optionally substituted benzoxaborol or benzoxaborinine. Benzoxaborol is sometimes referred to as benzoboroxol and the names may be used interchangeably (US 2014/0155305) The benzylic position of the boron atom in an optionally substituted benzoxaborole or benzoxaborinine stabilizes the empty p-orbital on the boron atom. Typically, the benzoxaborol or benzoxaborinine may be substituted with one or more of H, F, CI, NO₂, C₁-C₃alkyl, C₁-C₃haloalkyl, C₁-C₃alkoxy, C₃-C₆cycloalkyl, phenyl, and a five- to six-membered heteroaromatic ring comprising 1 to 3 heteroatoms selected from O, N and S.

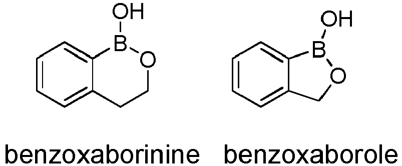

In one embodiment of this aspect of the invention, the method further comprises prior to the crosslinking step grafting said second glycosaminoglycan with said boronate hemiester, said boronate hemiester being a compound of Formula (I), 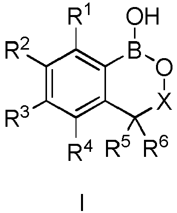 wherein
R¹ is selected from H, F, Cl, NO₂, C₁-C₃alkyl, C₁-C₃haloalkyl and C₁-C₃alkoxy; R², R³ and R⁴ are independently selected from H, F, Cl, C₁-C₃haloalkyl, NO₂, C₁-C₃alkoxy, C₁-C₃alkyl and a linker capable of binding covalently to said second glycosaminoglycan;
X is selected from CHR⁷ and a bond; and
R⁵, R⁶ and R⁷ are independently selected from H, C₁-C₄alkyl, C₃-C₆cycloalkyl, phenyl, and a five- to six-membered heteroaromatic ring comprising 1 to 3 heteroatoms selected from O, N and S, wherein one of R², R³ and R⁴ is a linker.

The benzylic position of the boron atom in a compound of Formula (I) or in an optionally substituted benzoxaborole or benzoxaborinine stabilizes the empty p-orbital on the boron atom. The linker in position R², R³ or R⁴ is the group binding a compound of Formula (I) to said second glycosaminoglycan and thus enables the grafting of said compound to said second glycosaminoglycan.

As used herein, the term "C₁-C₃haloalkyl" means both linear and branched chain saturated hydrocarbon groups, with 1 to 3 carbon atoms and with 1 to all hydrogenssubstituted by a halogen of different or same type. Examples of C₁-C₃haloalkyl groups include methyl substituted with 1 to 3 halogen atoms, ethyl substituted with 1 to 5 halogen atoms, and n-propyl or iso-propyl substituted with 1 to 7 halogen atoms.

As used herein, the term "C₁-C₃fluorooalkyl" means both linear and branched chain saturated hydrocarbon groups, with 1 to 3 carbon atoms and with 1 to all hydrogen atoms substituted by a fluorine atom. Examples of C₁-C₃fluoroalkyl groups include methyl substituted with 1 to 3 fluorine atoms, ethyl substituted with 1 to 5 fluorine atoms, and n-propyl or iso-propyl substituted with 1 to 7 fluorine atoms.

According to some embodiments, the glycosaminoglycan is selected from the group consisting of sulfated or non-sulfated glycosaminoglycans such as hyaluronan, chondroitin, chondroitin sulphate, heparan sulphate, heparosan, heparin, dermatan sulphate and keratan sulphate. According to some embodiments, the glycosaminoglycan is selected from the group consisting of hyaluronic acid, chondroitin and chondroitin sulfate, and mixtures thereof.

In one embodiment of this aspect of the invention, said first and said second glycosaminoglycans are hyaluronic acid. Hyaluronic acid (HA) is one of the most widely used biocompatible polymers for medical and cosmetic use. HA is a naturally occurring polysaccharide belonging to the group of glycosaminoglycans (GAGs). Hyaluronic acid consists of two alternating monosaccharides units, *N*-acetyl-D-glucosamine (GlcNAc) and D-glucuronic acid (GlcA), assembled by β(1→3) and β(1→4) glycosidic bonds, respectively. Hyaluronic acid and products derived from hyaluronic acid are widely used in the biomedical and cosmetic fields, for instance during viscosurgery and as a dermal filler.

Unless otherwise specified, the term "hyaluronic acid" encompasses all variants and combinations of variants of hyaluronic acid, hyaluronate or hyaluronan, of various chain lengths and charge states, as well as with various chemical modifications. That is, the term also encompasses the various hyaluronate salts of hyaluronic acid with various counter ions, such as sodium hyaluronate. The hyaluronic acid can be obtained from various sources of animal and non-animal origin. Sources of non-animal origin include yeast and preferably bacteria. The molecular weight of a single hyaluronic acid molecule is typically in the range of 0.1-10 kg/mol, but other molecular weights are possible. According to the invention, preferred molecular weights are in the range 50-3000 kg/mol, more preferably in the range 70-1000 kg/mol.

In one embodiment of this aspect of the invention, the molecular weight of the glycosaminoglycan is between 200-1500 kg/mol, preferably in the range 400-1100 kg/mol, more preferably 500-1000 kg/mol, more preferably 600-800 kg/mol. It has been experimentally observed that these ranges of molecular weights of the hyaluronic acid exhibit increasingly improved gel properties (e.g. G' and G"), when grafted with a boronate hemiester.

In one embodiment of this aspect of the invention, the degree of substitution of the glycosaminoglycans is 0.05-0.3, preferably 0.1-0.2.

The term "chondroitin" refers to glycosaminoglycans having a disaccharide repeating unit consisting of alternating non-sulfated D-glucuronic acid and N-acetyl-D-galactosamine moieties. For avoidance of doubt, the term "chondroitin" does not encompass any form of chondroitin sulfate.

The term "chondroitin sulfate" refers to glycosaminoglycans having a disaccharide repeating unit consisting of alternating D-glucuronic acid and N-acetyl-D-galactosamine moieties. The sulfate moiety can be present in various different positions. Preferred chondroitin sulfate molecules are chondroitin-4-sulfate and chondroitin-6-sulfate.

The chondroitin molecules can be obtained from various sources of animal and non-animal origin. Sources of non-animal origin include yeast and preferably bacteria. The molecular weight of a single chondroitin molecule is typically in the range of 1-500 kg/mol, but other molecular weights are possible.

In one embodiment of this aspect of the invention, said linker forms an amide bond or an ether bond to said second glycosaminoglycan. The grafting of the compound of Formula I to said second glycosaminoglycan may be done for example via an ether bond by reacting for example a hydroxy group of the backbone of the glycosaminoglycan with an epoxy functionality of said linker. The grafting of the compound of Formula I to said second glycosaminoglycan may also be done by using 4-(4,6-dimethoxy-1,3,5-triazin-2-yl)-4-methylmorpholinium chloride (DMTMM) to activate carboxylic groups on said second glycosaminoglycan and react the resulting species with an amine function of said linker to form a stable amide.

In one embodiment of this aspect of the invention, R² is a linker. When R² is used as the linker in the boronate hemiester a gel was obtained without the need to graft a sugar moiety to said first glycosaminoglycan.

In one embodiment of this aspect of the invention, said linker is H₂N-Y- or 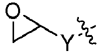 and forms an amide bond or an ether bond with said second glycosaminoglycan;
Y is selected from a bond and C₁-C₆alkylene in which one or two CH₂ are optionally replaced by a group selected from O, NH and phenylene, said C₁-C₆alkylene being optionally substituted with 1 to 12 R⁸; and
R⁸ is selected from F, Cl, C₁-C₃alkyl, C₁-C₃haloalkyl, phenyl, OH, C₁-C₃hydroxyalkyl, C₁-C₃alkoxy, NH₂, N-C₁-C₃alkylamino, N,N-Ci-C₄dialkylamino.

In one embodiment of this aspect of the invention, said linker is HR⁹N-Y- and forms an amide bond with said second glycosaminoglycan, wherein R⁹ is selected from hydrogen, C₁-C₃alkyl and C₁-C₃fluoroalkyl; and Y is a bond or an unsubstituted C₁-C₆alkylene. The grafting of the compound of Formula I to said second glycosaminoglycan may be done by using 4-(4,6-dimethoxy-1,3,5-triazin-2-yl)-4-methylmorpholinium chloride (DMTMM) to activate carboxylic groups on said second glycosaminoglycan and react the resulting species with an amine function (HR⁹N-Y-) of said linker to form a stable amide.

In one embodiment of this aspect of the invention, R⁹ is hydrogen.

In one embodiment of this aspect of the invention, the boronate hemiester is 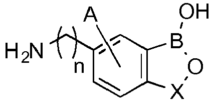
wherein A is selected from H, F, CF₃, NO₂, OCH₃ and CH₃;
n is selected from 0, 1, 2 and 3; and
X is selected from CH₂, CH₂-CH₂, CH-NC₅H₁₁ (CH-piperidine) and C(CH₃)₂.

In one embodiment of this aspect of the invention,
R¹, R³ and R⁴ are independently selected from H, F, OCH₃, CF₃ and CH₃;
R² is a linker;
said linker is H₂N-Y- and forms an amide bond with said second glycosaminoglycan;
Y is a bond or an unsubstituted C₁-C₃alkylene;
X is a bond; and
R⁵ and R⁶ are independently selected from H and C₁-C₃alkyl.

In one embodiment of this aspect of the invention, the boronate hemiester is selected from

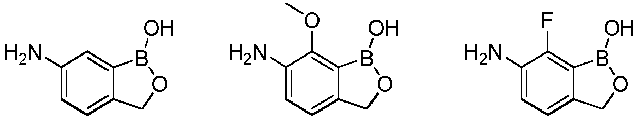

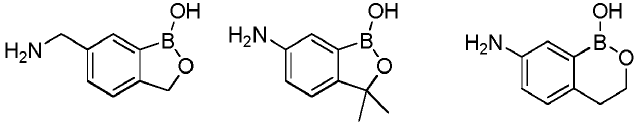

In one embodiment of this aspect of the invention, the boronate hemiester is selected from 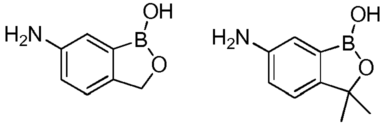 wherein the boronate hemiester is grafted to said second glycosaminoglycan by that the -NH₂ group of the boronate hemiester forms an amide with a backbone carboxylate group of said second glycosaminoglycan.

In one embodiment of this aspect of the invention, said boronate hemiester is

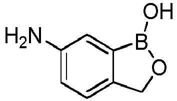

The compound above may be called 5-amino-2-hydroxymethylphenylboronic acid. According to the generated IUPAC name in Biovia DRAW 4.2, it should be named 1-hydroxy-3H-2,1-benzoxaborol-amine.

In one aspect of the invention, there is provided, crosslinked glycosaminoglycans produced according to the method aspect of the invention.

In one aspect of the invention, there is provided, use of a boronate hemiester in the manufacture of crosslinked glycosaminoglycans, the crosslinkage being via an alkoxyboronate ester anion formed between a backbone diol function of a first glycosaminoglycan and a boronate hemiester grafted to a second glycosaminoglycan. The use of a boronate hemiester in the manufacture of crosslinked glycosaminoglycans allows for binding directly to a diol function of the backbone of a glycosaminoglycan and to form a gel without the need for binding via a sugar moiety grafted onto a first glycosaminoglycan due to the high affinity towards diols of a boronate hemiester function. The use of a boronate hemiester to crosslink glycosaminoglycans, does not exclude that the glycosaminoglycans are further crosslinked. In WO 2014/072330, phenylboronic acid is used to crosslink hyaluronic acid, which does not allow for cross-linking to a backbone diol function of said first glycosaminoglycan (comparison in Example 3). The use of a boronate hemiester in the manufacture of crosslinked glycosaminoglycans according to the invention is thus simpler of crosslinking glycosaminoglycans, requiring less synthetic steps. As demonstrated in the appended examples a gel is formed by using a boronate hemiester grafted to a glycosaminoglycan without the need for grafting sugar moieties to a second glycosaminoglycan (example 1, 2 and 3). A glycosaminoglycan grafted with a boronate hemiester further provides self-healing properties to the obtained gel (see Figure 5, Example 3; Example 15, Figure 8). The use of a boronate hemiester in the manufacture of crosslinked glycosaminoglycans also provides crosslinked glycosaminoglycans that are easy to inject as the reversible bonds break when pushed through the syringe, and then quickly reform inside the body. The gels can be injected as preformed solids, because the solid gel can manage external damages and repair itself under a proper shear stress. Due to fast gelation kinetics after extrusion/injection, they recover their solid form almost immediately. Thus, before the gel reforms inside the body, the gel is malleable, until the reversible bonds reform. Thus, in one embodiment, the crosslinked glycosaminoglycans provides a self-healing gel.

In one embodiment of this aspect of the invention the boronate hemiester is a compound comprising a 5-6-membered cyclic boronate hemiester moiety.

In one embodiment of this aspect of the invention the boronate hemiester is a compound comprising an oxaborole or a oxaborinine moiety. A glycosaminoglycan grafted with a boroxole is shown in the appended examples to give rise to a gel.

In one embodiment of this aspect of the invention the boronate hemiester is an optionally substituted benzoxaborole or benzoxaborinine. The benzylic position of the boron atom in an optionally substituted benzoxaborol or benzoxaborinine stabilizes the empty p-orbital on the boron atom. Typically, the benzoxaborol or benzoxaborinine may be substituted with one or more of H, F, Cl, NO₂, C₁-C₃alkyl, C₁-C₃haloalkyl, C₁-C₃alkoxy, C₃-C₆cycloalkyl, phenyl, and a five- to six-membered heteroaromatic ring comprising 1 to 3 heteroatoms selected from O, N and S.

In one embodiment of this aspect of the invention, said boronate hemiester is a compound of Formula (II) 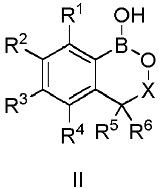 wherein
R¹ is selected from H, F, Cl, NO₂, C₁-C₃alkyl, C₁-C₃haloalkyl and C₁-C₃alkoxy;
R², R³ and R⁴ are independently selected from H, F, Cl, C₁-C₃haloalkyl, NO₂, C₁-C₃alkoxy, C₁-C₃alkyl and a linker capable of binding covalently to said second glycosaminoglycan;
X is selected from CHR⁷ and a bond; and
R⁵, R⁶ and R⁷ are independently selected from H, C₁-C₄alkyl, C₃-C₆cycloalkyl, phenyl, and a five- to six-membered heteroaromatic ring comprising 1 to 3 heteroatoms selected from O, N and S,
wherein one of R², R³ and R⁴ is a linker.

The benzylic position of the boron atom in a compound of Formula (I) or in an optionally substituted benzoxaborole or benzoxaborinine stabilizes the empty p-orbital on the boron atom. The linker in position R², R³ or R⁴ is the group binding a compound of Formula (II) to said second glycosaminoglycan and thus enables the grafting of said compound to said second glycosaminoglycan.

According to some embodiments, the glycosaminoglycan is selected from the group consisting of sulfated or non-sulfated glycosaminoglycans such as hyaluronan, chondroitin, chondroitin sulphate, heparan sulphate, heparosan, heparin, dermatan sulphate and keratan sulphate. According to some embodiments, the glycosaminoglycan is selected from the group consisting of hyaluronic acid, chondroitin and chondroitin sulfate, and mixtures thereof.

In one embodiment of this aspect of the invention, said glycosaminoglycans are hyaluronic acid.

In one embodiment of this aspect of the invention, the molecular weight of the glycosaminoglycan is between 200-1500 kg/mol, preferably in the range 400-1100 kg/mol, more preferably 500-1000 kg/mol, more preferably 600-800 kg/mol. It has been experimentally observed that these ranges of molecular weights of the hyaluronic acid exhibit increasingly improved gel properties (e.g. G' and G"), when grafted with a boronate hemiester.

In one embodiment of this aspect of the invention, the degree of substitution of the glycosaminoglycans is 0.05-0.3, preferable 0.1-0.2.

In one embodiment of this aspect of the invention,
said linker is capable of forming an amide bond or an ether bond to said second glycosaminoglycan. The grafting of the compound of Formula I to said second glycosaminoglycan may be done for example via an ether bond by reacting for example a hydroxy group of the backbone of the glycosaminoglycan with an epoxy functionality of said linker. The grafting of the compound of Formula I to said second glycosaminoglycan may also be done by using 4-(4,6-dimethoxy-1,3,5-triazin-2-yl)-4-methylmorpholinium chloride (DMTMM) to activate carboxylic groups on said second glycosaminoglycan and react the resulting species with an amine function of said linker to form a stable amide.

In one embodiment of this aspect of the invention, said linker is HR⁹N-Y- or 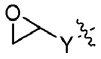 and capable of forming an amide bond or an ether bond with said second glycosaminoglycan, wherein R⁹ is selected from hydrogen, C₁-C₃alkyl and C₁-C₃fluoroalkyl; Y is selected from a bond and C₁-C₆alkylene in which one or two CH₂ are optionally replaced by a group selected from O, NH and phenylene, said C₁-C₆alkylene being optionally substituted with 1 to 12 R⁸; and
R⁸ is selected from F, Cl, C₁-C₃alkyl, C₁-C₃haloalkyl, phenyl, OH, C₁-C₃hydroxyalkyl, C₁-C₃alkoxy, NH₂, N-C₁-C₃alkylamino, N,N-Ci-C₄dialkylamino.

In one embodiment of this aspect of the invention, R² is a linker. When R² is used as the linker in the boronate hemiester a gel was obtained without the need to graft a sugar moiety to said first glycosaminoglycan.

In one embodiment of this aspect of the invention, said linker is HR⁹N-Y- and capable of forming an amide bond with said second glycosaminoglycan, wherein R⁹ is selected from hydrogen, C₁-C₃alkyl and C₁-C₃fluoroalkyl; and Y is a bond or an unsubstituted C₁-C₆alkylene. The grafting of the compound of Formula I to said second glycosaminoglycan may be done by using 4-(4,6-dimethoxy-1,3,5-triazin-2-yl)-4-methylmorpholinium chloride (DMTMM) to activate carboxylic groups on said second glycosaminoglycan and react the resulting species with an amine function (HR⁹N-Y-) of said linker to form a stable amide.

In one embodiment of this aspect of the invention, R⁹ is hydrogen.

In one embodiment of this aspect of the invention, the boronate hemiester is 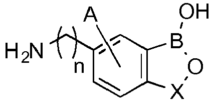
wherein A is selected from H, F, CF₃, NO₂, OCH₃ and CH₃;
n is selected from 0, 1, 2 and 3; and
X is selected from CH₂, CH₂-CH₂, CH-NC₅H₁₁ (CH-piperidine) and C(CH₃)₂.

In one embodiment of this aspect of the invention, R¹, R³ and R⁴ are independently selected from H, F, OCH₃, CF₃ and CH₃;
R² is a linker;
said linker is H₂N-Y- and capable of forming an amide bond with said second glycosaminoglycan;
Y is a bond or an unsubstituted C₁-C₃alkylene;
X is a bond; and
R⁵ and R⁶ are independently selected from H and C₁-C₃alkyl.

In one embodiment of this aspect of the invention, the boronate hemiester is selected from 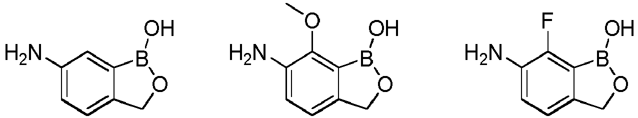 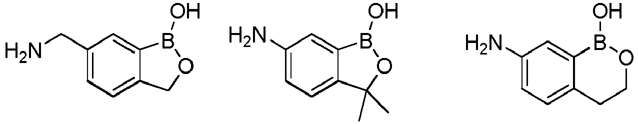 wherein the boronate hemiester is grafted to said second glycosaminoglycan by that the -NH₂ group of the boronate hemiester forms an amide with a backbone carboxylate group of said second glycosaminoglycan.

In one embodiment of this aspect of the invention, the boronate hemiester is selected from

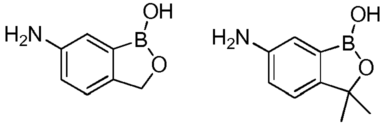

In one embodiment of this aspect of the invention, said boronate hemiester is

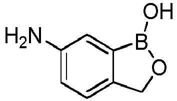

In one aspect of the invention, there is provided crosslinked glycosaminoglycans, wherein said glycosaminoglycans are crosslinked via an alkoxyboronate ester anion formed between a backbone diol function of a first glycosaminoglycan and a boronate hemiester grafted to a second glycosaminoglycan. Crosslinked glycosaminoglycans via an alkoxyboronate ester anion formed with a diol function of the backbone of said first glycosaminoglycan provides a gel with self-healing properties without the need for binding via a sugar moiety grafted onto a second glycosaminoglycan. Thus, a gel is provided with less modifications of the glycosaminoglycan. As demonstrated in the appended examples a gel is formed from a glycosaminoglycan grafted with a boronate hemiester (example 1, 2 and 3). Crosslinked glycosaminoglycan via an alkoxyboronate ester anion provides a gel with self-healing properties (see Figure 5, Example 3). The crosslinked glycosaminoglycans may optionally be further crosslinked. The crosslinked glycosaminoglycans are also easy to inject as the reversible bonds break when pushed through the syringe, and then quickly reform inside the body. The gels can be injected as preformed solids, because the solid gel can manage external damages and repair itself under a proper shear stress. Due to fast gelation kinetics after extrusion/injection, they recover their solid form almost immediately. Thus, before the gel reforms inside the body, the gel is malleable, until the reversible bonds reform. Thus, in one embodiment, the crosslinked glycosaminoglycans provides a self-healing gel.

In one embodiment of this aspect of the invention the boronate hemiester is a compound comprising a 5-6-membered cyclic boronate hemiester moiety.

In one embodiment of this aspect of the invention the boronate hemiester is a compound comprising an oxaborole or a oxaborinine moiety. A glycosaminoglycan grafted with a boroxole is shown in the appended examples to give rise to a gel.

In one embodiment of this aspect of the invention the boronate hemiester is an optionally substituted benzoxaborol or benzoxaborinine. The benzylic position of the boron atom in an optionally substituted benzoxaborol or benzoxaborinine stabilizes the empty p-orbital on the boron atom. Typically, the benzoxaborol or benzoxaborinine may be substituted with one or more of H, F, Cl, NO₂, C₁-C₃alkyl, C₁-C₃haloalkyl, C₁-C₃alkoxy, C₃-C₆cycloalkyl, phenyl, and a five- to six-membered heteroaromatic ring comprising 1 to 3 heteroatoms selected from O, N and S.

The present invention proposes new hydrogels:
- in which the glycosaminoglycan chains are only connected with reversible crosslinks and which are based on GAG-boroxole (BOR).

In one embodiment of this aspect of the invention, said crosslinked glycosaminoglycans has a structure of Formula (III) 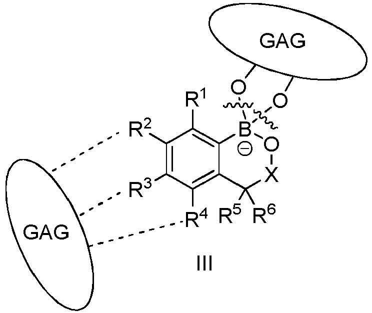 wherein
R¹ is selected from H, F, Cl, NO₂, C₁-C₃alkyl, C₁-C₃haloalkyl and C₁-C₃alkoxy; R², R³ and R⁴ are independently selected from H, F, Cl, C₁-C₃haloalkyl, NO₂, C₁-C₃alkoxy, C₁-C₃alkyl and a linker, said linker binding covalently to said second glycosaminoglycan;
X is selected from CHR⁷ and a bond;
R⁵, R⁶ and R⁷ are independently selected from H, C₁-C₄alkyl, C₃-C₆cycloalkyl, phenyl, and a five- to six-membered heteroaromatic ring comprising 1 to 3 heteroatoms selected from O, N and S; and
one of R², R³ and R⁴ is a linker.

The present disclosure provides new hydrogel products and related advantageous processes for preparing hydrogels made of crosslinked glycosaminoglycan (GAG) molecules having reversible linkages, and uses thereof. Glycosaminoglycans are negatively charged heteropolysaccharide chains which have a capacity to absorb large amounts of water. In the hydrogel products according to the disclosure, the crosslinked glycosaminoglycan molecule is the swellable polymer which provides the gel properties.

The polysaccharide according to the present disclosure is preferably a glycosaminoglycan (GAG). According to some embodiments, the glycosaminoglycan is selected from the group consisting of sulfated or non-sulfated glycosaminoglycans such as hyaluronan, chondroitin, chondroitin sulphate, heparan sulphate, heparosan, heparin, dermatan sulphate and keratan sulphate. According to some embodiments, the glycosaminoglycan is selected from the group consisting of hyaluronic acid, chondroitin and chondroitin sulfate, and mixtures thereof. According to some embodiments, the glycosaminoglycan is hyaluronic acid.

In one embodiment of this aspect of the invention, said glycosaminoglycans are hyaluronic acid.

In one embodiment of this aspect of the invention, the molecular weight of the glycosaminoglycan is between 200-1500 kg/mol, preferably in the range 400-1100 kg/mol, more preferably 500-1000 kg/mol, more preferably 600-800 kg/mol. It has been experimentally observed that these ranges of molecular weights of the hyaluronic acid, when crosslinked via an alkoxyboronate ester anion, exhibit increasingly improved gel properties (e.g. G' and G").

In one embodiment of this aspect of the invention, the degree of substitution of the glycosaminoglycans is 0.05-0.3, preferable 0.1-0.2.

Hyaluronic acid (HA) is one of the most widely used biocompatible polymers for medical and cosmetic use. HA is a naturally occurring polysaccharide belonging to the group of glycosaminoglycans (GAGs). Hyaluronic acid consists of two alternating monosaccharides units, *N*-acetyl-D-glucosamine (GlcNAc) and D-glucuronic acid (GlcA), assembled by β(1→3) and β(1→4) glycosidic bonds, respectively. Hyaluronic acid and products derived from hyaluronic acid are widely used in the biomedical and cosmetic fields, for instance during viscosurgery and as a dermal filler.

Unless otherwise specified, the term "hyaluronic acid" encompasses all variants and combinations of variants of hyaluronic acid, hyaluronate or hyaluronan, of various chain lengths and charge states, as well as with various chemical modifications. That is, the term also encompasses the various hyaluronate salts of hyaluronic acid with various counter ions, such as sodium hyaluronate. The hyaluronic acid can be obtained from various sources of animal and non-animal origin. Sources of non-animal origin include yeast and preferably bacteria. The molecular weight of a single hyaluronic acid molecule is typically in the range of 0.1-10 kg/mol, but other molecular weights are possible. According to the invention, preferred molecular weights are in the range 50-3000 kg/mol, more preferably in the range 70-1000 kg/mol

The term "chondroitin" refers to glycosaminoglycans having a disaccharide repeating unit consisting of alternating non-sulfated D-glucuronic acid and N-acetyl-D-galactosamine moieties. For avoidance of doubt, the term "chondroitin" does not encompass any form of chondroitin sulfate.

The term "chondroitin sulfate" refers to glycosaminoglycans having a disaccharide repeating unit consisting of alternating D-glucuronic acid and N-acetyl-D-galactosamine moieties. The sulfate moiety can be present in various different positions. Preferred chondroitin sulfate molecules are chondroitin-4-sulfate and chondroitin-6-sulfate.

The chondroitin molecules can be obtained from various sources of animal and non-animal origin. Sources of non-animal origin include yeast and preferably bacteria. The molecular weight of a single chondroitin molecule is typically in the range of 1-500 kg/mol, but other molecular weights are possible.

The term "crosslinked glycosaminoglycans" or "crosslinked glycosaminoglycan molecules" refers herein to glycosaminoglycans comprising, typically covalent, crosslinks between the glycosaminoglycan molecule chains, which creates a continuous network of glycosaminoglycan molecules held together by the crosslinks.

The crosslinked glycosaminoglycan product is preferably biocompatible. This implies that no, or only very mild, immune response occurs in the treated individual. That is, no or only very mild undesirable local or systemic effects occur in the treated individual.

The crosslinked product according to the disclosure is a gel, or a hydrogel. That is, it can be regarded as a water-insoluble, but substantially dilute crosslinked system of glycosaminoglycan molecules when subjected to a liquid, typically an aqueous liquid.

The crosslinked glycosaminoglycan gel can be simply obtained from solutions of HA-BOR in a physiological buffer.
Alternatively, the crosslinked glycosaminoglycan gels can present the form of gel particles. The gel particles have an average size in the range of 0.01-5 mm, preferably 0.1-0.8 mm, such as 0.2-0.5 mm or 0.5-0.8 mm.

Due to its significant liquid content, the gel product is structurally flexible and similar to natural tissue, which makes it very useful as a scaffold in tissue engineering and for tissue augmentation. It is also useful for treatment of soft tissue disorder and for corrective or aesthetic treatment. It is preferably used as an injectable formulation.

The hydrogel product may be present in an aqueous solution, but it may also be present in dried or precipitated form, e.g. in ethanol.

The hydrogel product is preferably injectable.

The hyaluronic acid can be obtained from various sources of animal and non-animal origin. Sources of non-animal origin include yeast and preferably bacteria. The molecular weight of a single hyaluronic acid molecule is typically in the range of 0.1-10 kg/mol, but other molecular weights are possible.

In certain embodiments the concentration of said hyaluronic acid is in the range of 1 to 100 mg/ml. In some embodiments the concentration of said hyaluronic acid is in the range of 2 to 50 mg/ml. In specific embodiments the concentration of said hyaluronic acid is in the range of 5 to 30 mg/ml or in the range of 10 to 30 mg/ml. In certain embodiments, the hyaluronic acid is permanently crosslinked. Crosslinked hyaluronic acid comprises crosslinks between the hyaluronic acid chains, which creates a continuous network of hyaluronic acid molecules which is held together by reversible covalent crosslinks or reversible covalent crosslinks in addition to permanent covalent crosslinks.

Crosslinking of hyaluronic acid may be achieved by modification with a boroxole derivative. The degree of substitution (DS) of these HA-conjugates can be varied in a range from 0.05 to 0.30 in order to tune the rheological behavior of the gels. The degree of substitution may be measured on the substituted polymer glycosaminoglycans by NMR.

A typical application of the resulting hydrogel product involves the preparation of injectable formulations for treatment of soft tissue disorders, including, but not limited to, corrective and aesthetic treatments.

In one embodiment of this aspect of the invention, said linker forms an amide bond or an ether bond with said second glycosaminoglycan;
The boronate hemiester of Formula I may be grafted to said second glycosaminoglycan for example via an ether bond by reacting for example a hydroxy group of the backbone of the glycosaminoglycan with an epoxy functionality of said linker. The boronate hemiester of Formula I may be grafted to said second glycosaminoglycan via an amide between an amine function of said linker and a carboxylate group on said second glycosaminoglycan. This may be done by using 4-(4,6-dimethoxy-1,3,5-triazin-2-yl)-4-methylmorpholinium chloride (DMTMM) to activate carboxylic groups on said second glycosaminoglycan and react the resulting species with an amine of said linker.

In one embodiment of this aspect of the invention, said linker is -NR⁹-Y-
or -O-Y- and forms an amide bond or an ether bond with said second glycosaminoglycan, wherein R⁹ is selected from hydrogen, C₁-C₃alkyl and C₁-C₃fluoroalkyl;
Y is selected from a bond and C₁-C₆alkylene in which one or two CH₂ are optionally replaced by a group selected from O, NH and phenylene, said C₁-C₆alkylene being optionally substituted with 1 to 12 R⁸; and
R⁸ is selected from F, Cl, C₁-C₃alkyl, C₁-C₃haloalkyl, phenyl, OH, C₁-C₃hydroxyalkyl, C₁-C₃alkoxy, NH₂, N-C₁-C₃alkylamino, N,N-Ci-C₄dialkylamino.

In one embodiment of this aspect of the invention, R² is a linker. When R² is the linker in the boronate hemiester, a gel of crosslinked glycosaminoglycans was obtained without the need to graft a sugar moiety to said first glycosaminoglycan.

In one embodiment of this aspect of the invention, said linker is -NR⁹-Y- and forms an amide bond with said second glycosaminoglycan, wherein R⁹ is selected from hydrogen, C₁-C₃alkyl and C₁-C₃fluoroalkyl; and wherein Y is a bond or an unsubstituted C₁-C₆alkylene. The boronate hemiester of Formula I may be grafted to said second glycosaminoglycan via an amide between an amine function of said linker and a carboxylate group on said second glycosaminoglycan. This may be done by using 4-(4,6-dimethoxy-1,3,5-triazin-2-yl)-4-methylmorpholinium chloride (DMTMM) to activate carboxylic groups on said second glycosaminoglycan and react the resulting species with an amine of said linker.

In one embodiment of this aspect of the invention, the boronate hemiester is 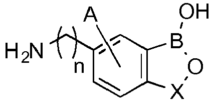
wherein A is selected from H, F, CF₃, NO₂, OCH₃ and CH₃;
n is selected from 0, 1, 2 and 3; and
X is selected from CH₂, CH₂-CH₂, CH-NC₅H₁₁ (CH-piperidine) and C(CH₃)₂.

In one embodiment of this aspect of the invention,
R¹, R³ and R⁴ are independently selected from H, F, OCH₃, CF₃ and CH₃; R² is a linker;
said linker is -HN-Y- and forms an amide bond with said second glycosaminoglycan;
Y is a bond or unsubstituted C₁-C₃alkylene;
X is a bond or CH₂; and
R⁵ and R⁶ are independently selected from H and C₁-C₃alkyl.
X has two connections and the term CH₂ thus means methylene or -CH₂-.

In one embodiment of this aspect of the invention,
R¹ is selected from H, F and OCH₃;
R² is a linker;
R³ and R⁴ are hydrogen;
said linker is -HN- and forms an amide bond with said second glycosaminoglycan;
Y is a bond or an unsubstituted methylene;
X is a bond or CH₂; and
R⁵ and R⁶ are independently selected from H and C₁-C₃alkyl.

In one embodiment of this aspect of the invention, the boronate hemiester is selected from

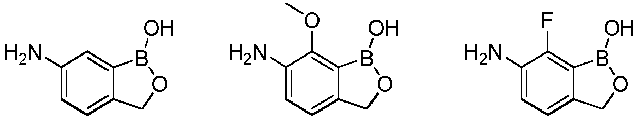

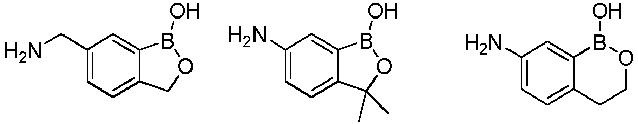

In one embodiment of this aspect of the invention, the boronate hemiester is selected from 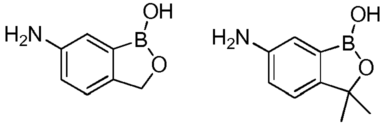 wherein the boronate hemiester is grafted to said second glycosaminoglycan by that the -NH₂ group of the boronate hemiester forms an amide with a backbone carboxylate group of said second glycosaminoglycan.

In one embodiment of this aspect of the invention, said crosslinked glycosaminoglycans having a structure of Formula (IV)

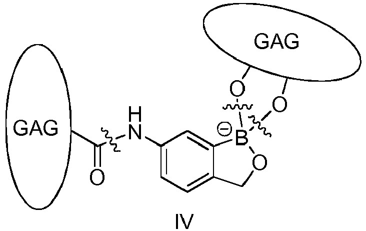

In one aspect of the invention, there is provided a polymer composition comprising crosslinked glycosaminoglycans according to the invention and an aqueous buffer. The buffer may stabilize the gel and make it particularly robust towards changes of pH. The buffer is typically a HEPES buffer. According to related aspects, the present disclosure also provides use of the hydrogel product as a medicament, such as in the treatment of soft tissue disorders. There is provided a method of treating a patient suffering from a soft tissue disorder by administering to the patient a therapeutically effective amount of the hydrogel product. There is also provided a method of providing corrective or aesthetic treatment to a patient by administering to the patient a therapeutically effective amount of the hydrogel product.

According to other aspects illustrated herein, there is provided a hydrogel product obtained by the inventive method for use as a medicament.

According to other aspects illustrated herein, there is provided a hydrogel product obtained by the inventive method for use in the treatment of soft tissue disorders.

According to other aspects illustrated herein, there is provided the use of a hydrogel product obtained by the inventive method for the manufacture of a medicament for treatment of soft tissue disorders.

According to other aspects illustrated herein, there is provided a method of treating a patient suffering from a soft tissue disorder by administering to the patient a therapeutically effective amount of a hydrogel product obtained by the inventive method.

According to other aspects illustrated herein, there is provided a method of providing corrective or aesthetic treatment to a patient by administering to the patient a therapeutically effective amount of a hydrogel product obtained by the inventive method.

According to other aspects illustrated herein, there is provided a method of cosmetically treating skin, which comprises administering to the skin a hydrogel product obtained by the inventive method.

Other aspects and preferred embodiments of the present invention will be evident from the appended examples and the appended claims.

The term "molecular weight" as used herein in connection with various polymers, e.g. polysaccharides, refers to the weight average molecular weight, M_{w}, of the polymers, which is well defined in the scientific literature. The weight average molecular weight can be determined by, e.g., static light scattering, small angle neutron scattering, X-ray scattering, and sedimentation velocity. The unit of the molecular weight for polymers is g/mol. The person skilled in the art realizes that the present invention by no means is limited to the preferred embodiments described herein. On the contrary, many modifications and variations are possible within the scope of the appended claims. Additionally, variations to the disclosed embodiments can be understood and effected by the skilled person in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

One object of the invention concerns novel hydrogels synthesized by reversible crosslinking of boronate ester bonds based on benzoboroxole modified hyaluronic acid (HA-BOR).

The derivatives of benzoboroxole acid selected consist of but, are not limited to compound of formula (I): 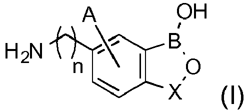
With A = H, F, CF₃, NO₂, OCH₃, CH₃
n= 0, 1, 2 or 3
X = CH₂, CH₂-CH₂, CH-NC₅H₁₁ (CH-piperidine), C(CH₃)₂

The preferred derivatives of benzoboroxole is the benzoboroxole of formula (II)

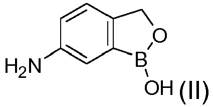

HA-Boroxole obtained is a compound of formula (III)

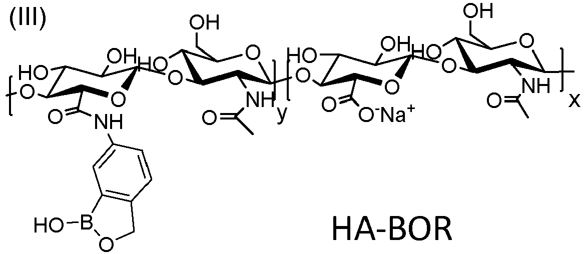

Or

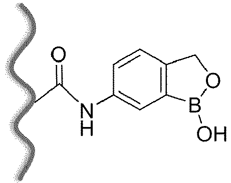

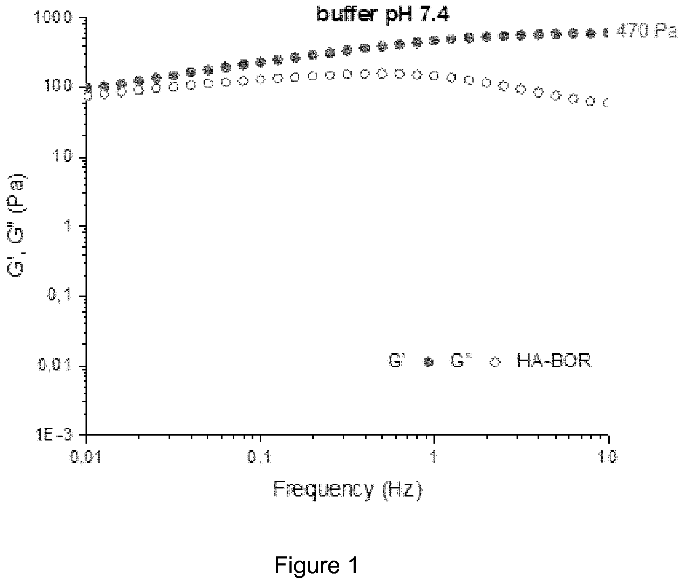

Boronate ester bonds are formed between benzoboroxole and diol groups on HA chain. The product obtained can be represented as below (formula IV). Gels behavior has been demonstrated by rheological analysis (Fig 1).

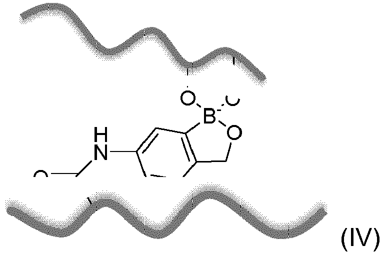

In one aspect of the invention, there is provided a polymer composition comprising glycosaminoglycans (GAG) crosslinked by reversible boronate ester bonds.

In one embodiment of the invention, the glycosaminoglycan is hyaluronic acid (HA).

In one embodiment of the invention, the polymer composition comprises boroxole (BOR) modified hyaluronic acid (HA) polymer grafted at the carboxylate group comprising boroxole. In other words, the polymer composition comprises hyaluronic acid grafted with a boroxole to a carboxylate group.

### Brief description of the drawings

Figure 1: Rheological analysis: measurement of G' and G" for HA-BOR (15 g/l in 0.01 M HEPES buffer with 0.15 M NaCl, pH 7.4).
Figure 2: Rheological analysis: measurement of G' and G" for HA-BOR with HA M_{w} 500 kg/mol (HA500), [PS] of 15 g/L (HA-BOR derivative solubilized in ultrapure water at 30 g/L, followed by addition of 0.02 M HEPES buffer containing 0.3 M NaCl pH 7.4)
Figure 3: Rheological analysis: measurement of G' and G" for HA-BOR with HA M_{w} 600 kg/mol (HA600), [PS] of 15 g/L (HA-BOR derivative solubilized in ultrapure water at 30 g/L, followed by addition of 0.02 M HEPES buffer containing 0.3 M NaCl pH 7.4)
Figure 4: Rheological analysis: measurement of G' and G" for HA-BOR with HA M_{w} 1000 kg/mol (HA1000), [PS] of 15 g/L (HA-BOR derivative solubilized in ultrapure water at 30 g/L, followed by addition of 0.02 M HEPES buffer containing 0.3 M NaCl pH 7.4).
Figure 5. Self-healing behavior of a HA-BOR hydrogel: application of gradually increasing stress values from 1800 to 2100 Pa for 2 min, intercalated with periods of application of a strain fixed at 5% for 3 min (frequency fixed at 1 Hz).
Figure 6: Gel obtained with HA-BOR.
Figure 7: Rheological analysis: measurement of G' and G" for HA-BOR and HA-DMABOR gels with HA M_{w} 600 kg/mol (HA600), [PS] of 15 g/L (HA derivatives solubilized in ultrapure water at 30 g/L, followed by addition of 0.02 M HEPES buffer containing 0.3 M NaCl pH 7.4).
Figure 8: Recovery of *G*'and *G*" as a function of time post-extrusion of a HA-DMABOR gel (M_{w} = 600 kg/mol) through a 27 gauge needle.

The following terms and characteristics will be used in the examples and results shown. The definitions are the one hereafter:
**Mw** - Molecular Weight : The mass average molecular mass
**DS-** Degree of Substitution The term "degree of substitution" (DS) as used herein in connection with various polymers, e.g. polysaccharides, refers to the average number of substituting group per repeating disaccharide unit.
**[PS]** - The polysaccharide concentration (g/l).
**G':** storage (elastic) modulus (in Pa)
**G"**: loss (viscous) modulus (in Pa)
**G' 1Hz:** storage modulus (in Pa) measured at a frequency of 1 Hz
**G" 1Hz:** loss modulus (in Pa) measured at a frequency of 1 Hz
**Gel-like behavior:** G'> G" within the whole range of frequency covered (0.01-10 Hz)
**Viscoelastic behavior:** viscous (G'<G") and elastic (G'>G") behavior observed within the range of frequency covered (0.01-10 Hz).
**The IUPAC names** of the benzoboroxol derivatives in example 4-11 are generated using Biovia DRAW 4.2.

### EXAMPLES

Without desiring to be limited thereto, the present invention will in the following be illustrated by way of examples.

### Example 1 : Synthesis of HA-BOR

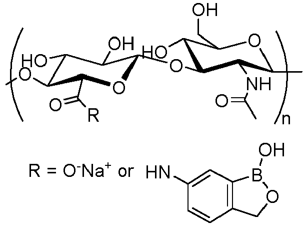

The amine-acid coupling agent 4-(4,6-dimethoxy-1,3,5-triazin-2-yl)-4-methylmorpholinium chloride (DMTMM) was dissolved in 1 mL of water and was added to a solution of native HA in a mixture of water/DMF (3/2, v/v). The concentration of HA in the reaction medium depends on its molar mass (Table 1, M_{w} HA). Then, 5-amino-2-hydroxymethylphenylboronic acid hydrochloride (i.e. 1-hydroxy-3H-2,1-benzoxaborol-amine hydrochloride or BOR) solubilized in 1 mL of water was added to the reaction medium. The pH was adjusted to 6.5 using 0.5 M HCI or NaOH and the reaction was kept under stirring at room temperature for 24 h. The product was purified by diafiltration with ultrapure water and was recovered by freeze-drying. The degree of substitution (DS) was determined by ¹H NMR (DS_{NMR}), and was also estimated from the reaction kinetics performed using 2,4,6-Trinitrobenzene Sulfonic Acid (DS_{TNBS}). This method consisted in quantifying the free primary amines in the reaction medium as a function of time.

### Results:

Table 1 summarizes the concentration of HA (C_{HA}), the DMTMM/HA and BOR/HA molar ratios used for the syntheses with different M_{w} HA, as well as the DS and the yields of HA-BOR conjugates.

**Table 1. Syntheses of HA-BOR.**

| **M_{w} HA (Kg/mol)** | **C_{HA} (g/L)** | **DMTMM/HA molar ratio** | **BOR/HA molar ratio** | **DS_{NMR}^{a}** | **DS_{TNBS}** | **Yield** (**%)^{b}** |
|---|---|---|---|---|---|---|
| 100 | 3 | 1 | 0.16 | 0.12 | 0.14 | 85 |
| 500 | 2 | 1 | 0.14 (or 0.25) | 0.1 (or 0.2) | 0.12 (or 0.22) | 91 (or 77) |
| 600 | 2 | 1 | 0.14 (or 0.25) | 0.11 (or 0.2) | 0.13 (or 0.2) | 75 (or 72) |
| 1000 | 1 | 1 | 0.14 (or 0.26) | 0.1 (or 0.2) | 0.14 (or 0.23) | 84 (or 60) |

| | | | | | | |
|---|---|---|---|---|---|---|
| ^{a}DS by ¹H NMR: 10 % of accuracy. ^{b}HA-BOR yield: calculation considering the DS_{NMR}. | | | | | | |

**HA-BOR (HA-1-hydroxy-3H-2,1-benzoxaborol-amine):** ¹H NMR (400 MHz, D₂O) δ_{H} (ppm) 4.55 (H-1 from *N*-acetylglucosamine unit), 4.25 (H-1 from glucuronic acid), 3.9-3.1 (H-2, H-3, H-4, H-5, H-6 protons of HA), 2.08 (C**H₃**-CO from HA), 7.95 (s, 1H, NH-C-C**H**-C-B from Ph), 7.72 (m, 1H, C-CH-C**H**-C-C-B from Ph), 7.55 (m, 1H, C-CH-C**H**-C-C-B from Ph), 5.13 (s, 2H, C**H₂**-O-B).

### Example 2 : Preparation of HA-benzoboroxole (HA-BOR) gel

HA-BOR gels were prepared by solubilizing 1-hydroxy-3H-2,1-benzoxaborolamine (the HA-BOR derivative) in 0.01 M HEPES buffer with 0.15 M NaCl at physiological pH ([PS] = 15 g/L).

### Results:

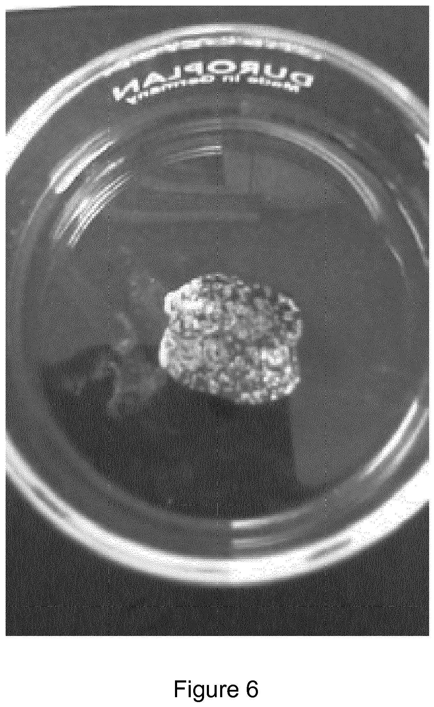

Characteristics of the obtained HA-BOR hydrogels are summarized in Table 1. Boronate ester bonds are formed between benzoboroxole and HA hydroxy groups. Gels behavior has been demonstrated by rheological analysis (Fig 1). Surprinsingly, when coupling HA chains with benzoboroxole only, obtained hydrogels present good gel behaviour (Fig.6).

**Table 2 : Characteristics of HA-BOR hydrogel ([PS] = 15 g/L).**

| **HA-boronic acid derivative** | **DS HA-boronic acid derivative** | **Mw HA (kg/mol)** | **G' 1Hz (Pa)** | **G" 1Hz (Pa)** | **Rheological behavior** |
|---|---|---|---|---|---|
| HA-BOR | 0.11 | 600 | 470 | 145 | Gel-like behavior |
| HA-BOR | 0.2 | 600 | 420 | 130 | Gel-like behavior |
| HA-BOR | 0.1 | 1000 | 56 | 36 | Viscolelastic behavior |

### Example 3: Comparison of HA-BOR gel to HA-PBA gel and native HA gel

### HA-BOR gel preparation:

HA-1-hydroxy-3H-2,1-benzoxaborol-amine (HA-BOR derivative) was solubilized in ultrapure water (pH 5-6) at 30 g/L for 24 h under continuous stirring at 4 °C, followed by addition of 0.02M HEPES buffer containing 0.3M NaCl pH 7.4 (final [PS] = 15 g/L).

### Results:

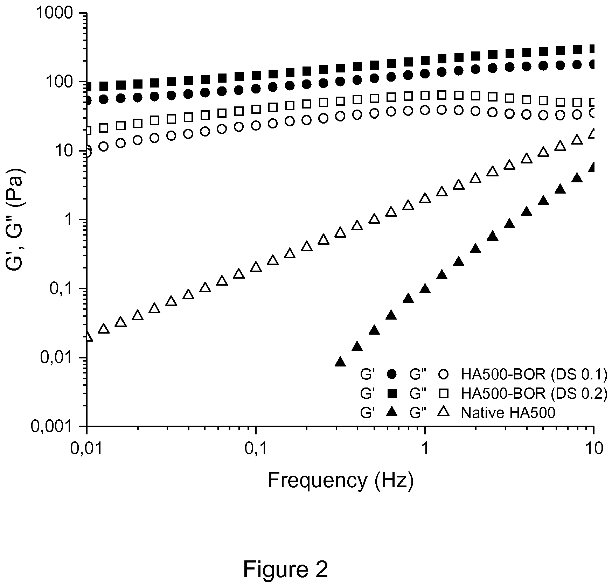

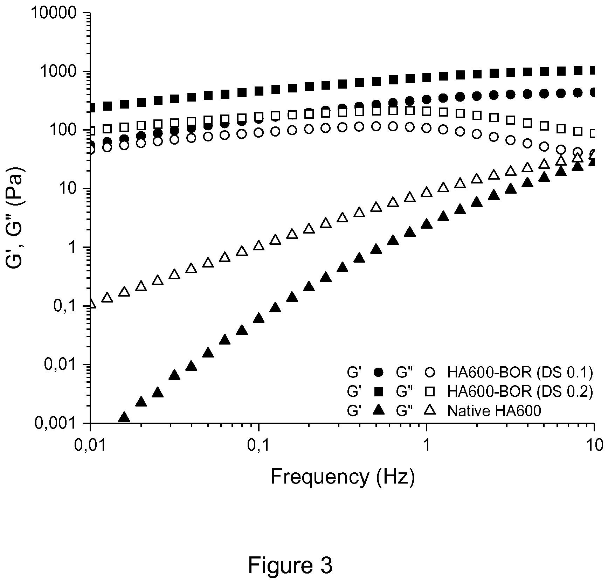

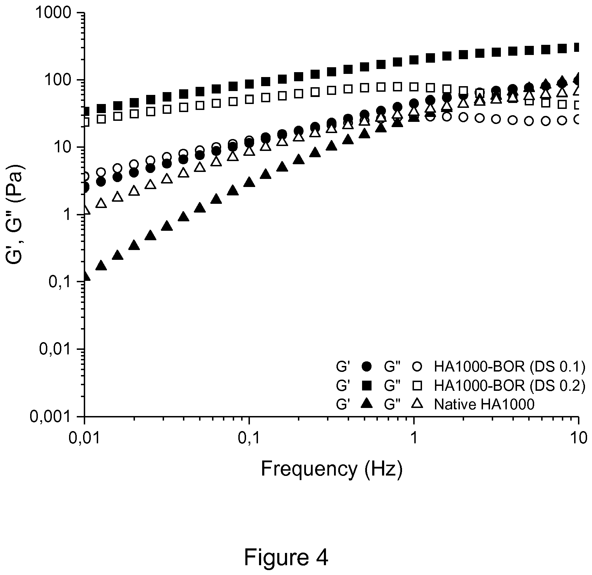

Within 8 h of stirring at 4 °C, a final gel was obtained with a polymer concentration of 15 g/L and pH 7. Gels prepared using HA-BOR with M_{w} of 1000 kg/mol may require a longer time of solubilization (24 to 48 h). Characteristics of the resulting gels or viscous solutions are are shown in Table 3. Plotted measurements of G' and G" for HA-BOR gels with different molecular weights and degrees of substitution, compared to native HA are shown in figures 2, 3 and 4.

Self-healing properties of a dynamic gel of HA-BOR (CHA = 15 g/L) at 25 °C were investigated by, while measuring G' and G", applying successive stress values from 1800 to 2100 Pa for 2 min. These were intercalated with short time periods in which low stress values (corresponding to 5 % strain) were applied for 3 min. This experiment demonstrated the stress recovery of the HA-BOR gel after 4 cycles of stress-induced breakdowns. Large stress (from 1800 to 2100 Pa) inverted the values of G' (filled circles) and G" (empty circles), indicating breakage of crosslinks and conversion to solution state. G' was recovered under a small strain (5 %) within few seconds. The obtained HA-BOR showed self-healing properties, see figure 5.

### Synthesis of HA-PBA:

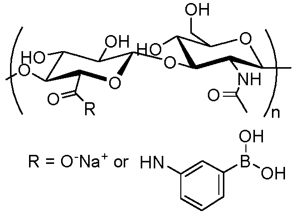

3-aminophenylboronic acid hemisulfate salt (APBA) dissolved in 1 mL of water was added to a solution of native HA in a mixture of water/DMF (3/2, v/v), in the presence of 4-(4,6-dimethoxy-1,3,5-triazin-2-yl)-4-methylmorpholinium chloride (DMTMM) as an amine-acid coupling agent. A concentration of 2 g/L was used for the sample HA M_{w} 600 kg/mol. The pH was adjusted to 6.5 using 0.5 M HCI or NaOH and the reaction was kept under stirring at room temperature for 24 h. The product was purified by diafiltration with ultrapure water and was recovered by freeze-drying. The degree of substitution (DS) of HA-PBA was determined by ¹H NMR (0.14 ± 0.01). HA-PBA was obtained with a yield of 78 % (calculated considering its DS).

**HA-PBA:** ¹H NMR (400 MHz, D₂O) δ_{H} (ppm) 4.55 (H-1 from *N-*acetylglucosamine unit), 4.25 (H-1 from glucuronic acid), 3.9-3.1 (H-2, H-3, H-4, H-5, H-6 protons of HA), 2.08 (C**H₃**-CO from HA), 7.93 (s, 1H, NH-C-C**H**-C-B from Ph), 7.7 (m, 2H, C-C**H**-CH-C**H**-C-B from Ph), 7.55 (m, 1H, C-CH-CH-CH-C-B from Ph).

### HA-PBA and native HA samples preparation:

HA-PBA or native HA was solubilized in ultrapure water (pH 5-6) at 30 g/L for 24 h under continuous stirring at 4 °C, followed by addition of 0.02M HEPES buffer containing 0.3M NaCl pH 7.4. The solutions were stirred during 8 h at 4 °C.

Result: The characteristics of the resulting samples are shown in Table 3.

**Table 3 : Characteristics of obtained samples ([PS] = 15 g/L).**

| **HA derivative** | **DS HA derivative** | **Mw HA (kg/mol)** | **G'1Hz (Pa)** | **G" 1Hz (Pa)** | **Rheological behavior** |
|---|---|---|---|---|---|
| HA-BOR | 0.1 | 100 | 0.043 | 0.44 | Viscous |
| HA-BOR | 0.1 | 500 | 160 | 38 | Gel |
| HA-BOR | 0.2 | 500 | 204 | 63 | Gel |
| HA-BOR | 0.1 | 600 | 330 | 108 | Gel |
| HA-BOR | 0.2 | 600 | 800 | 210 | Gel |
| HA-BOR | 0.1 | 1000 | 45 | 29 | Viscoelastic |
| HA-BOR | 0.2 | 1000 | 198 | 78 | Gel |
| HA-PBA | 0.15 | 600 | 5.65 | 5.89 | Viscoelastic |
| Native HA | - | 500 | 0.05 | 1.3 | Viscous |
| Native HA | - | 500 | 0.1 | 1.96 | Viscous |
| Native HA | - | 600 | 2 | 8 | Viscous |
| Native HA | - | 1000 | 27 | 33 | Viscoelastic |

### Example 4 : Synthesis of HA-1-hydroxy-7-methoxy-3H-2,1-benzoxaborol-6-amine

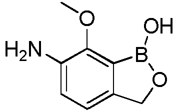

Example 4 is performed according to Example 1, but using 1-hydroxy-7-methoxy-3H-2,1-benzoxaborol-6-amine hydrochloride as the BOR derivative instead of 1-hydroxy-3H-2,1-benzoxaborol-amine hydrochloride.

### Example 5 : HA-1-hydroxy-7-methoxy-3H-2,1-benzoxaborol-6-amine gel preparation:

HA-1-hydroxy-7-methoxy-3H-2,1-benzoxaborol-6-amine is solubilized in ultrapure water (pH 5-6) at 30 g/L for 24 h under continuous stirring at 4 °C, followed by addition of 0.02M HEPES buffer containing 0.3M NaCl pH 7.4.

Another set of gels are prepared according to example 2, but using HA-1-hydroxy-7-methoxy-3H-2,1-benzoxaborol-6-amine instead of HA-BOR.

### Example 6 : Synthesis of HA-7-fluoro-1-hydroxy-3H-2,1-benzoxaborol-6-amine

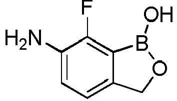

Example 6 is performed according to Example 2, but using 7-fluoro-1-hydroxy-3H-2,1-benzoxaborol-6-amine hydrochloride as the BOR derivative instead of 1-hydroxy-3H-2,1-benzoxaborol-amine hydrochloride.

### Example 7 : HA-7-fluoro-1-hydroxy-3H-2,1-benzoxaborol-6-amine gel preparation:

HA-7-fluoro-1-hydroxy-3H-2,1-benzoxaborol-6-amine is solubilized in ultrapure water (pH 5-6) at 30 g/L for 24 h under continuous stirring at 4 °C, followed by addition of 0.02M HEPES buffer containing 0.3M NaCl pH 7.4.

Another set of gels are prepared according to example 2, but using HA-7-fluoro-1-hydroxy-3H-2,1-benzoxaborol-6-amine instead of HA-BOR.

### Example 8 : Synthesis of HA-(1-hydroxy-3H-2,1-benzoxaborol-6-yl)methanamine

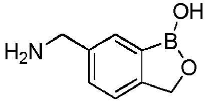

Example 8 is performed according to Example 1, but using (1-hydroxy-3H-2,1-benzoxaborol-6-yl)methanamine hydrochloride as the BOR derivative instead of 1-hydroxy-3H-2,1-benzoxaborol-amine hydrochloride.

### Example 9 : HA-(1-hydroxy-3H-2,1-benzoxaborol-6-yl)methanamine gel preparation:

HA-(1-hydroxy-3H-2,1-benzoxaborol-6-yl)methanamine is solubilized in ultrapure water (pH 5-6) at 30 g/L for 24 h under continuous stirring at 4 °C, followed by addition of 0.02M HEPES buffer containing 0.3M NaCl pH 7.4.

Another set of gels are prepared according to example 2, but using HA-(1-hydroxy-3H-2,1-benzoxaborol-6-yl)methanamine instead of HA-BOR.

### Example 10 : Synthesis of HA-1-hydroxy-3,3-dimethyl-2,1-benzoxaborol-6-amine

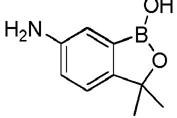

Example 10 is performed according to Example 1, but using 1-hydroxy-3,3-dimethyl-2,1-benzoxaborol-6-amine hydrochloride as the BOR derivative instead of 1-hydroxy-3H-2,1-benzoxaborol-amine hydrochloride.

### Example 11 : HA-1-hydroxy-3,3-dimethyl-2,1-benzoxaborol-6-amine gel preparation:

HA-1-hydroxy-3,3-dimethyl-2,1-benzoxaborol-6-amine is solubilized in ultrapure water (pH 5-6) at 30 g/L for 24 h under continuous stirring at 4 °C, followed by addition of 0.02M HEPES buffer containing 0.3M NaCl pH 7.4.

Another set of gels are prepared according to example 2, but using HA-1-hydroxy-3,3-dimethyl-2,1-benzoxaborol-6-amine instead of HA-BOR.

### Example 12 : Synthesis of HA-1-hydroxy-3,4-dihydro-2,1 benzoxaborinin-7-amine

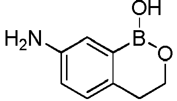

Example 10 is performed according to Example 1, but using 1-hydroxy-3,4-dihydro-2,1-benzoxaborinin-7-amine hydrochloride as the BOR derivative instead of 1-hydroxy-3H-2,1-benzoxaborol-amine hydrochloride.

### Example 13 : HA-1-hydroxy-3,4-dihydro-2,1-benzoxaborinin-7-amine gel preparation:

HA-1-hydroxy-3,4-dihydro-2,1-benzoxaborinin-7-amine is solubilized in ultrapure water (pH 5-6) at 30 g/L for 24 h under continuous stirring at 4 °C, followed by addition of 0.02M HEPES buffer containing 0.3M NaCl pH 7.4.

Another set of gels are prepared according to example 2, but using HA-1-hydroxy-3,4-dihydro-2,1-benzoxaborinin-7-amine instead of HA-BOR.

### Example 14: Synthesis of HA-1-hydroxy-3,3-dimethyl-2,1-benzoxaborol-6-amine

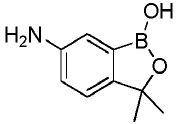

Example 14 was performed according to Example 1, but using 1-hydroxy-3,3-dimethyl-2,1-benzoxaborol-6-amine hydrochloride (DMABOR) as the BOR derivative instead of 1-hydroxy-3H-2,1-benzoxaborol-amine hydrochloride. The molecular weight of the Hyaluronic acid was 600 kg/mol.

### Example 15: HA-1-hydroxy-3,3-dimethyl-2,1-benzoxaborol-6-amine gel preparation

HA-1-hydroxy-3,3-dimethyl-2,1-benzoxaborol-6-amine (HA-DMABOR) was solubilized in ultrapure water (pH 5-6) at 30 g/L for 24 h under continuous stirring at 4 °C, followed by addition of 0.02M HEPES buffer containing 0.3M NaCl pH 7.4 (final [PS] = 15 g/L).

Gels were prepared according to example 2, but using HA-1-hydroxy-3,3-dimethyl-2,1-benzoxaborol-6-amine (HA-DMABOR) instead of HA-BOR.

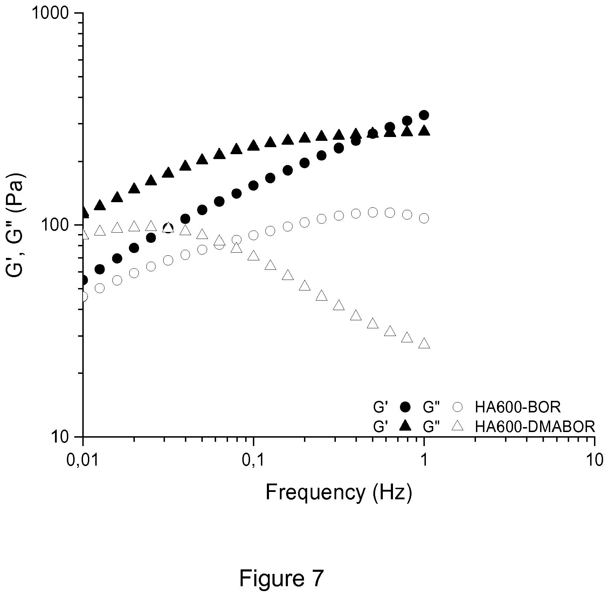

Result: Characteristics of the obtained HA-DMABOR hydrogel are summarized in Table 4, in comparison with HA-BOR gel. Boronate ester bonds are formed between DMABOR and HA hydroxyl groups. Gels behavior has been demonstrated by rheological analysis (Fig 7). These hydrogel further exhibited self-healing properties. Consequently, they can be injected as preformed solids, because the solid gel can manage external damages and repair itself under a proper shear stress. Due to fast gelation kinetics after extrusion/injection, they recover their solid form immediately. As an example, Figure 8 shows the variation of G' and G" as a function of time immediately after injection of a HA-DMABOR gel (in 0.01M HEPES/0.15M NaCl buffer pH 7.5, at a [PS] = 15 g/L) through a 27 gauge needle. From this Figure, it can be seen that the sample recovered into a solid gel quasi-instantaneously.

**Table 4: Characteristics of obtained gels ([PS] = 15 q/L).**

| **HA derivative** | **DS HA derivative** | **Mw HA (kg/mol)** | **G' 1Hz (Pa)** | **G" 1Hz (Pa)** | **Rheological behavior** |
|---|---|---|---|---|---|
| HA-BOR | 0.1 | 600 | 330 | 108 | Gel |
| HA-DMABOR | 0.1 | 600 | 270 | 27 | Gel |

## Claims

1. Crosslinked glycosaminoglycans, wherein said glycosaminoglycans are crosslinked via an alkoxyboronate ester anion formed between a backbone diol function of a first glycosaminoglycan and a boronate hemiester grafted to a second glycosaminoglycan.

2. Crosslinked glycosaminoglycans according to claim 1, wherein said crosslinked glycosaminoglycans has a structure of Formula (III) 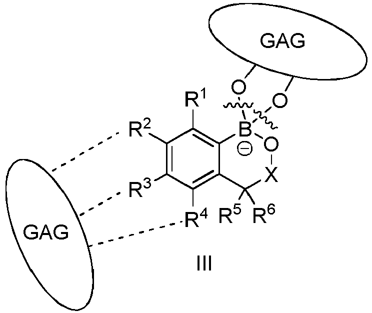 wherein
R¹ is selected from H, F, Cl, NO₂, C₁-C₃alkyl, C₁-C₃haloalkyl and C₁-C₃alkoxy;
R², R³ and R⁴ are independently selected from H, F, Cl, C₁-C₃haloalkyl, NO₂, C₁-C₃alkoxy, C₁-C₃alkyl and a linker, said linker binding covalently to said second glycosaminoglycan;
X is selected from CHR⁷ and a bond;
R⁵, R⁶ and R⁷ are independently selected from H, C₁-C₄alkyl, C₃-C₆cycloalkyl, phenyl, and a five- to six-membered heteroaromatic ring comprising 1 to 3 heteroatoms selected from O, N and S; and one of R², R³ and R⁴, such as R², is a linker.

3. Crosslinked glycosaminoglycans according to claim 1 or claim 2, wherein said glycosaminoglycans are hyaluronic acid.

4. Crosslinked glycosaminoglycans according to claim 2 or 3, wherein said linker is -NR⁹-Y- or -O-Y- and forms an amide bond or an ether bond with said second glycosaminoglycan, wherein R⁹ is selected from hydrogen, C₁-C₃alkyl and C₁-C₃fluoroalkyl;
Y is selected from a bond and C₁-C₆alkylene in which one or two CH₂ are optionally replaced by a group selected from O, NH and phenylene, said C₁-C₆alkylene being optionally substituted with 1 to 12 R⁸; and
R⁸ is selected from F, Cl, C₁-C₃alkyl, C₁-C₃haloalkyl, phenyl, OH, C₁-C₃hydroxyalkyl, C₁-C₃alkoxy, NH₂, N-C₁-C₃alkylamino, N,N-Ci-C₄dialkylamino.

5. Crosslinked glycosaminoglycans according to any one of claims 2 to 4, wherein
R¹, R³ and R⁴ are independently selected from H, F, OCH₃, CF₃ and CH₃;
R² is a linker;
said linker is -HN-Y- and forms an amide bond with said second glycosaminoglycan;
Y is a bond or an unsubstituted C₁-C₃alkylene;
X is a bond or CH₂; and
R⁵ and R⁶ are independently selected from H and C₁-C₃alkyl.

6. Crosslinked glycosaminoglycans according to claim 1, wherein said boronate hemiester is selected from 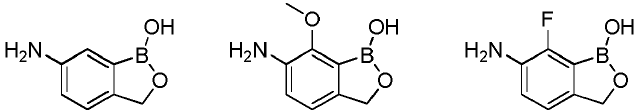 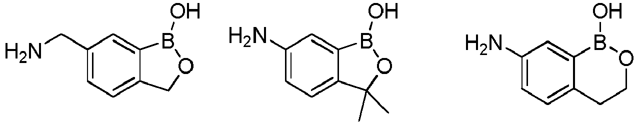 wherein the boronate hemiester is grafted to said second glycosaminoglycan by that the -NH₂ group of the boronate hemiester forms an amide with a backbone carboxylate group of said second glycosaminoglycan.

7. A method of cross-linking a first glycosaminoglycan having a backbone diol function and a second glycosaminoglycan being grafted with a boronate hemiester, comprising crosslinking said first glycosaminoglycan with said second glycosaminoglycan by forming an alkoxyboronate ester anion linkage between the boronate hemiester of the second glycosaminoglycan with the backbone diol function of said first glycosaminoglycan.

8. A method according to claim 7, further comprising the step grafting said second glycosaminoglycan with said boronate hemiester, said boronate hemiester being a compound of Formula (I), 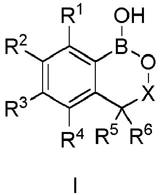 wherein
R¹ is selected from H, F, Cl, NO₂, C₁-C₃alkyl, C₁-C₃haloalkyl and C1-C₃alkoxy;
R², R³ and R⁴ are independently selected from H, F, Cl, C₁-C₃haloalkyl, NO₂, C₁-C₃alkoxy, C₁-C₃alkyl and a linker binding covalently to said second glycosaminoglycan;
X is selected from CHR⁷ and a bond; and
R⁵, R⁶ and R⁷ are independently selected from H, C₁-C₄alkyl, C₃-C₆cycloalkyl, phenyl, and a five- to six-membered heteroaromatic ring comprising 1 to 3 heteroatoms selected from O, N and S, wherein one of R², R³ and R⁴, such as R², is a linker, thereby providing said second glycosaminoglycan being grafted with a boronate hemiester.

9. A method according to claim 7 or claim 8, wherein said first and said second glycosaminoglycans are hyaluronic acid.

10. A method according to claim 8 or claim 9, wherein said linker is H₂N-Y- or 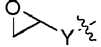 and forms an amide bond or an ether bond to said second glycosaminoglycan;
Y is selected from a bond and C₁-C₆alkylene in which one or two CH₂ are optionally replaced by a group selected from O, NH and phenylene, said C₁-C₆alkylene being optionally substituted with 1 to 12 R⁸; and
R⁸ is selected from F, Cl, C₁-C₃alkyl, C₁-C₃haloalkyl, phenyl, OH, C₁-C₃hydroxyalkyl, C₁-C₃alkoxy, NH₂, N-C₁-C₃alkylamino, N,N-Ci-C₄dialkylamino.

11. A method according to any one of claims 8 to 10, wherein R¹, R³ and R⁴ are independently selected from H, F, OCH₃, CF₃ and CH₃;
R² is a linker;
said linker is H₂N-Y- and forms an amide bond with said second glycosaminoglycan;
Y is a bond or an unsubstituted C₁-C₃alkylene;
X is a bond or CH₂; and
R⁵ and R⁶ are independently selected from H and C₁-C₃alkyl.

12. A method according to claim 7, wherein said boronate hemiester is selected from 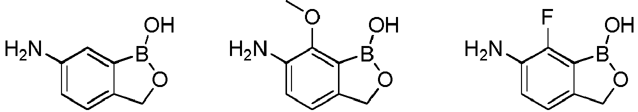 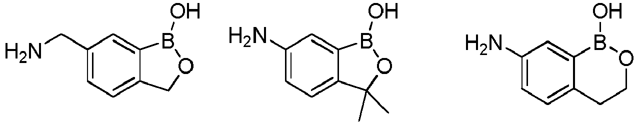 wherein the boronate hemiester is grafted to said second glycosaminoglycan by that the -NH₂ group of the boronate hemiester forms an amide with a backbone carboxylate group of said second glycosaminoglycan.

13. Use of a boronate hemiester in the manufacture of crosslinked glycosaminoglycans, such as crosslinked hyaluronic acid, the crosslinkage being via an alkoxyboronate ester anion formed between a backbone diol function of a first glycosaminoglycan and a boronate hemiester grafted to a second glycosaminoglycan.

14. Use according to claim 13, wherein said boronate hemiester is a compound of Formula (II) 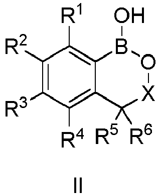 wherein
R¹ is selected from H, F, Cl, NO₂, C₁-C₃alkyl, C₁-C₃haloalkyl and C₁-C₃alkoxy;
R², R³ and R⁴ are independently selected from H, F, Cl, C₁-C₃haloalkyl, NO₂, C₁-C₃alkoxy, C₁-C₃alkyl and a linker capable of binding covalently to said second glycosaminoglycan;
X is selected from CHR⁷ and a bond; and
R⁵, R⁶ and R⁷ are independently selected from H, C₁-C₄alkyl, C₃-C₆cycloalkyl, phenyl, and a five- to six-membered heteroaromatic ring comprising 1 to 3 heteroatoms selected from O, N and S, wherein one of R², R³ and R⁴ is a linker, such as R² is a linker.

15. Polymer composition comprising crosslinked glycosaminoglycans according to any one of claims 1 to 6 and an aqueous buffer.

## Patentansprüche

1. Vernetzte Glykosaminoglykane, wobei die Glykosaminoglykane über ein Alkoxyboronatesteranion vernetzt sind, das zwischen einer Rückgratdiolfunktion eines ersten Glykosaminoglykans und einem an ein zweites Glykosaminoglykan gepfropften Boronathemiester gebildet wurde.

2. Vernetzte Glykosaminoglykane nach Anspruch 1, wobei die vernetzten Glykosaminoglykane eine Struktur der Formel (III) aufweisen: 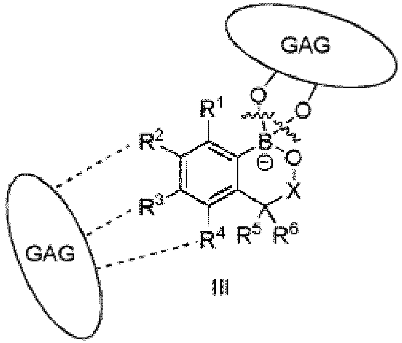 wobei
R¹ aus H, F, Cl, NO₂, C₁-C₃-Alkyl, C₁-C₃-Haloalkyl und C₁-C₃-Alkoxy ausgewählt ist; R², R³ und R⁴ unabhängig aus H, F, Cl, C₁-C₃-Haloalkyl, NO₂, C₁-C₃-Alkoxy, C₁-C₃-Alkyl und einem Linker ausgewählt sind, wobei der Linker kovalent an das zweite Glykosaminoglykan bindet;
X aus CHR⁷ und einer Bindung ausgewählt ist;
R⁵, R⁶ und R⁷ unabhängig aus H, C₁-C₄-Alkyl, C₃-C₆-Cycloalkyl, Phenyl und einem 5- bis 6-gliedrigen heteroaromatischen Ring ausgewählt sind, der 1 bis 3 Heteroatome umfasst, die aus O, N und S ausgewählt sind; und
eines von R², R³ und R⁴ wie z. B. R² ein Linker ist.

3. Vernetzte Glykosaminoglykane nach Anspruch 1 oder 2, wobei die Glykosaminoglykane Hyaluronsäure sind.

4. Vernetzte Glykosaminoglykane nach Anspruch 2 oder 3, wobei der Linker -NR⁹-Y- oder -O-Y- ist und eine Amidbindung oder eine Etherbindung mit dem zweiten Glykosaminoglykan bildet, wobei R⁹ aus Wasserstoff, C₁-C₃-Alkyl und C₁-C₃-Fluoralkyl ausgewählt ist;
Y aus einer Bindung und C₁-C₆-Alkylen ausgewählt ist, wobei ein oder zwei CH₂ optional durch eine Gruppe ersetzt sind, die aus O, NH und Phenylen ausgewählt ist, wobei das C₁-C₆-Alkylen optional mit 1 bis 12 R⁸ substituiert ist; und
R⁸ aus F, Cl, C₁-C₃-Alkyl, C₁-C₃-Haloalkyl, Phenyl, OH, C₁-C₃-Hydroxyalkyl, C₁-C₃-Alkoxy, NH₂, N-C₁-C₃-Alkylamino, N,N-C₁-C₄-Dialkylamino ausgewählt ist.

5. Vernetzte Glykosaminoglykane nach einem der Ansprüche 2 bis 4, wobei:
R¹, R³ und R⁴ unabhängig aus H, F, OCH₃, CF₃ und CH₃ ausgewählt sind;
R² ein Linker ist;
der Linker -HN-Y ist und eine Amidbindung mit dem zweiten Glykosaminoglykan bildet;
Y eine Bindung oder ein unsubstituiertes C₁-C₃-Alkylen ist;
X eine Bindung oder CH₂ ist; und
R⁵ und R⁶ unabhängig aus H und C₁-C₃-Alkyl ausgewählt sind.

6. Vernetzte Glykosaminoglykane nach Anspruch 1, wobei der Boronathemiester ausgewählt ist aus: 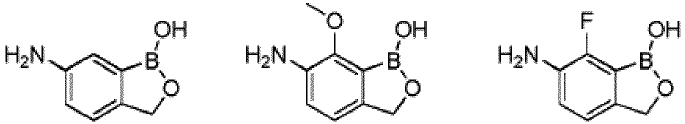 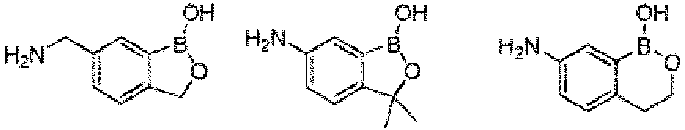 wobei der Boronathemiester dadurch an das zweite Glykosaminoglykan gepfropft wird, dass die -NH₂-Gruppe des Boronathemiesters ein Amid mit einer Rückgratcarboxylatgruppe des zweiten Glykosaminoglykans bildet.

7. Verfahren zum Vernetzen eines ersten Glykosaminoglykans mit einer Rückgratdiolfunktion und eines zweiten Glykosaminoglykans, das mit einem Boronathemiester gepfropft ist, das umfasst:
Vernetzen des ersten Glykosaminoglykans mit dem zweiten Glykosaminoglykan durch Bilden einer Alkoxyboronatesteranionverknüpfung zwischen dem Boronathemiester des zweiten Glykosaminoglykans mit der Rückgratdiolfunktion des ersten Glykosaminoglykans.

8. Verfahren nach Anspruch 7, das ferner den Schritt des Pfropfens des zweiten Glykosaminoglykans mit dem Boronathemiester umfasst, wobei der Boronathemiester eine Verbindung der Formel (I) ist: 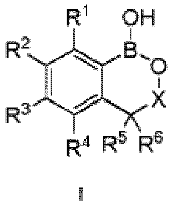 wobei
R¹ aus H, F, Cl, NO₂, C₁-C₃-Alkyl, C₁-C₃-Haloalkyl und C₁-C₃-Alkoxy ausgewählt ist; R², R³ und R⁴ unabhängig aus H, F, Cl, C₁-C₃-Haloalkyl, NO₂, C₁-C₃-Alkoxy, C₁-C₃-Alkyl und einem Linker ausgewählt sind, der kovalent an das zweite Glykosaminoglykan bindet;
X aus CHR⁷ und einer Bindung ausgewählt ist; und
R⁵, R⁶ und R⁷ unabhängig aus H, C₁-C₄-Alkyl, C₃-C₆-Cycloalkyl, Phenyl und einem 5- bis 6-gliedrigen heteroaromatischen Ring ausgewählt sind, der 1 bis 3 Heteroatome umfasst, die aus O, N und S ausgewählt sind,
wobei eines von R², R³ und R⁴ wie z. B. R² ein Linker ist, wodurch das zweite Glykosaminoglykan bereitgestellt wird, das mit einem Boronathemiester gepfropft ist.

9. Verfahren nach Anspruch 7 oder 8, wobei das erste und das zweite Glykosaminoglykan Hyaluronsäure sind.

10. Verfahren nach Anspruch 8 oder 9, wobei: der Linker H₂N-Y- oder 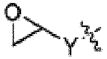 ist und eine Amidbindung oder eine Etherbindung an das zweite Glykosaminoglykan bildet;
Y aus einer Bindung und C₁-C₆-Alkylen ausgewählt ist, wobei ein oder zwei CH₂ optional durch eine Gruppe ersetzt sind, die aus O, NH und Phenylen ausgewählt ist, wobei das C₁-C₆-Alkylen optional mit 1 bis 12 R⁸ substituiert ist; und
R⁸ aus F, Cl, C₁-C₃-Alkyl, C₁-C₃-Haloalkyl, Phenyl, OH, C₁-C₃-Hydroxyalkyl, C₁-C₃-Alkoxy, NH₂, N-C₁-C₃-Alkylamino, N,N-C₁-C₄-Dialkylamino ausgewählt ist.

11. Verfahren nach einem der Ansprüche 8 bis 10, wobei:
R¹, R³ und R⁴ unabhängig aus H, F, OCH₃, CF₃ und CH₃ ausgewählt sind;
R² ein Linker ist;
der Linker H₂N-Y- ist und eine Amidbindung mit dem zweiten Glykosaminoglykan bildet;
Y eine Bindung oder ein unsubstituiertes C₁-C₃-Alkylen ist;
X eine Bindung oder CH₂ ist; und
R⁵ und R⁶ unabhängig aus H und C₁-C₃-Alkyl ausgewählt sind.

12. Verfahren nach Anspruch 7, wobei der Boronathemiester ausgewählt ist aus: 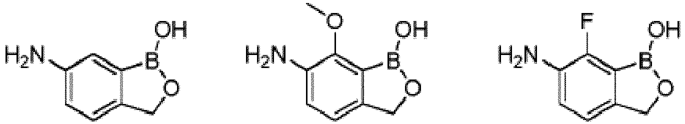 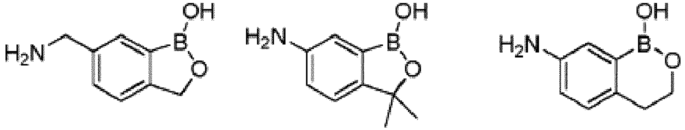 wobei der Boronathemiester dadurch an das zweite Glykosaminoglykan gepfropft wird, dass die -NH₂-Gruppe des Boronathemiesters ein Amid mit einer Rückgratcarboxylatgruppe des zweiten Glykosaminoglykans bildet.

13. Verwendung eines Boronathemiesters bei der Herstellung von vernetzten Glykosaminoglykanen wie z. B. einer vernetzten Hyaluronsäure, wobei die Vernetzung über ein Alkoxyboronatesteranion erfolgt, das zwischen einer Rückgratdiolfunktion eines ersten Glykosaminoglykans und einem an ein zweites Glykosaminoglykan gepfropften Boronathemiester gebildet wurde.

14. Verwendung nach Anspruch 13, wobei der Boronathemiester eine Verbindung der Formel (II) ist: 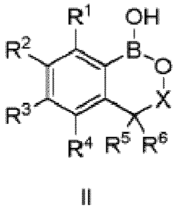 wobei
R¹ aus H, F, Cl, NO₂, C₁-C₃-Alkyl, C₁-C₃-Haloalkyl und C₁-C₃-Alkoxy ausgewählt ist;
R², R³ und R⁴ unabhängig aus H, F, Cl, C₁-C₃-Haloalkyl, NO₂, C₁-C₃-Alkoxy, C₁-C₃-Alkyl und einem Linker ausgewählt sind, der in der Lage ist, kovalent an das zweite Glykosaminoglykan zu binden;
X aus CHR⁷ und einer Bindung ausgewählt ist;
R⁵, R⁶ und R⁷ unabhängig aus H, C₁-C₄-Alkyl, C₃-C₆-Cycloalkyl, Phenyl und einem 5- bis 6-gliedrigen heteroaromatischen Ring ausgewählt sind, der 1 bis 3 Heteroatome umfasst, die aus O, N und S ausgewählt sind; und
wobei eines von R², R³ und R⁴ ein Linker ist, z. B. R² ein Linker ist.

15. Polymerzusammensetzung, die vernetzte Glykosaminoglykane nach einem der Ansprüche 1 bis 6 und einen wässrigen Puffer umfasst.

## Revendications

1. Glycosaminoglycanes réticulés, lesquels glycosaminoglycanes sont réticulés via un anion ester alcoxyboronate formé entre une fonction diol de charpente d'un premier glycosaminoglycane et un hémiester boronate greffé à un deuxième glycosaminoglycane.

2. Glycosaminoglycanes réticulés selon la revendication 1, lesquels glycosaminoglycanes réticulés ont une structure de formule (III) 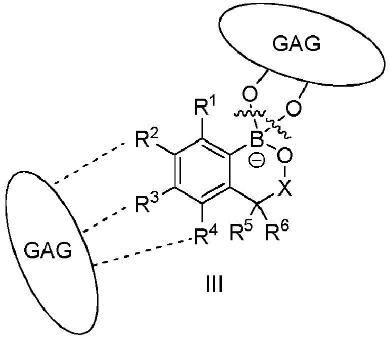 dans laquelle
R¹ est choisi parmi H, F, Cl, NO₂, alkyle en C₁ à C₃, halogénoalkyle en C₁ à C₃ et alcoxy en C₁ à C₃ ;
R², R³ et R⁴ sont indépendamment choisis parmi H, F, Cl, halogénoalkyle en C₁ à C₃, NO₂, alcoxy en C₁ à C₃, alkyle en C₁ à C₃ et un lieur, ledit lieur se liant de manière covalente audit deuxième glycosaminoglycane ;
X est choisi parmi CHR⁷ et une liaison ;
R⁵, R⁶ et R⁷ sont indépendamment choisis parmi H, alkyle en C₁ à C₄, cycloalkyle en C₃ à C₆, phényle, et un cycle hétéroaromatique à cinq à six chaînons comprenant 1 à 3 hétéroatomes choisis parmi O, N et S ; et
l'un de R², R³ et R⁴, tel que R², est un lieur.

3. Glycosaminoglycanes réticulés selon la revendication 1 ou la revendication 2, lesquels glycosaminoglycanes sont l'acide hyaluronique.

4. Glycosaminoglycanes réticulés selon la revendication 2 ou 3, dans lesquels ledit lieur est -NR⁹-Y- ou -O-Y- et forme une liaison amide ou une liaison éther avec ledit deuxième glycosaminoglycane, et dans lesquels
R⁹ est choisi parmi un hydrogène, alkyle en C₁ à C₃ et fluoroalkyle en C₁ à C₃ ;
Y est choisi parmi une liaison et un alkylène en C₁ à C₆ dans lequel un ou deux CH₂ sont éventuellement remplacés par un groupe choisi parmi O, NH et phénylène, ledit alkylène en C₁ à C₆ étant éventuellement substitué par 1 à 12 R⁸ ; et
R⁸ est choisi parmi F, Cl, alkyle en C₁ à C₃, halogénoalkyle en C₁ à C₃, phényle, OH, hydroxyalkyle en C₁ à C₃, alcoxy en C₁ à C₃, NH₂, N-alkylamino en C₁ à C₃, N,N-di(alkyle en C₁ à C₄)amino.

5. Glycosaminoglycanes réticulés selon l'une quelconque des revendications 2 à 4, dans lesquels
R¹, R³ et R⁴ sont indépendamment choisis parmi H, F, OCH₃, CF₃ et CH₃ ;
R² est un lieur ;
ledit lieur est -NH-Y- et forme une liaison amide avec ledit deuxième glycosaminoglycane ;
Y est une liaison ou un alkylène en C₁ à C₃ non substitué ;
X est une liaison ou CH₂ ; et
R⁵ et R⁶ sont indépendamment choisis parmi H et alkyle en C₁ à C₃.

6. Glycosaminoglycanes réticulés selon la revendication 1, dans lesquels ledit hémiester boronate est choisi parmi 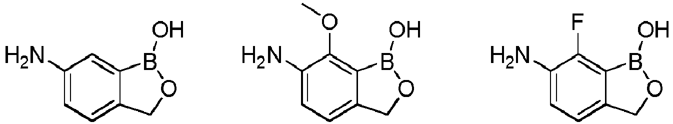 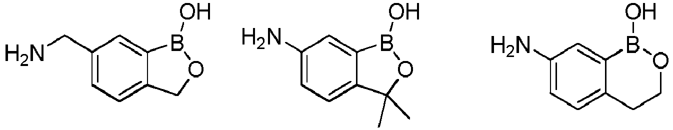 et dans lequel l'hémiester boronate est greffé audit deuxième glycosaminoglycane du fait que le groupe -NH₂ de l'hémiester boronate forme un amide avec un groupe carboxylate de charpente dudit deuxième glycosaminoglycane.

7. Procédé de réticulation d'un premier glycosaminoglycane ayant une fonction diol de charpente et d'un deuxième glycosaminoglycane qui est greffé avec un hémiester boronate, comprenant la réticulation dudit premier glycosaminoglycane avec ledit deuxième glycosaminoglycane par formation d'une liaison anionique de type ester alcoxyboronate entre l'hémiester boronate du deuxième glycosaminoglycane et la fonction diol de charpente dudit premier glycosaminoglycane.

8. Procédé selon la revendication 7, comprenant en outre l'étape de greffage dudit deuxième glycosaminoglycane avec ledit hémiester boronate, ledit hémiester boronate étant un composé de formule (I), 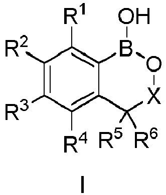 dans laquelle
R¹ est choisi parmi H, F, Cl, NO₂, alkyle en C₁ à C₃, halogénoalkyle en C₁ à C₃ et alcoxy en C₁ à C₃ ;
R², R³ et R⁴ sont indépendamment choisis parmi H, F, Cl, halogénoalkyle en C₁ à C₃, NO₂, alcoxy en C₁ à C₃, alkyle en C₁ à C₃ et un lieur se liant de manière covalente audit deuxième glycosaminoglycane ;
X est choisi parmi CHR⁷ et une liaison ; et
R⁵, R⁶ et R⁷ sont indépendamment choisis parmi H, alkyle en C₁ à C₄, cycloalkyle en C₃ à C₆, phényle, et un cycle hétéroaromatique à cinq à six chaînons comprenant 1 à 3 hétéroatomes choisis parmi O, N et S,
dans lequel l'un de R², R³ et R⁴, tel que R², est un lieur, ce qui donne ainsi ledit deuxième glycosaminoglycane qui est greffé avec un hémiester boronate.

9. Procédé selon la revendication 7 ou la revendication 8, dans lequel ledit premier et ledit deuxième glycosaminoglycanes sont l'acide hyaluronique.

10. Procédé selon la revendication 8 ou la revendication 9, dans lequel ledit lieur est H₂N-Y- ou 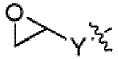 et forme une liaison amide ou une liaison éther avec ledit deuxième glycosaminoglycane ;
Y est choisi parmi une liaison et un alkylène en C₁ à C₆ dans lequel un ou deux CH₂ sont éventuellement remplacés par un groupe choisi parmi O, NH et phénylène, ledit alkylène en C₁ à C₆ étant éventuellement substitué par 1 à 12 R⁸ ; et
R⁸ est choisi parmi F, Cl, alkyle en C₁ à C₃, halogénoalkyle en C₁ à C₃, phényle, OH, hydroxyalkyle en C₁ à C₃, alcoxy en C₁ à C₃, NH₂, N-alkylamino en C₁ à C₃, N,N-di(alkyle en C₁ à C₄)amino.

11. Procédé selon l'une quelconque des revendications 8 à 10, dans lequel R¹, R³ et R⁴ sont indépendamment choisis parmi H, F, OCH₃, CF₃ et CH₃ ;
R² est un lieur ;
ledit lieur est -NH-Y- et forme une liaison amide avec ledit deuxième glycosaminoglycane ;
Y est une liaison ou un alkylène en C₁ à C₃ non substitué ;
X est une liaison ou CH₂ ; et
R⁵ et R⁶ sont indépendamment choisis parmi H et alkyle en C₁ à C₃.

12. Procédé selon la revendication 7, dans lequel ledit hémiester boronate est choisi parmi 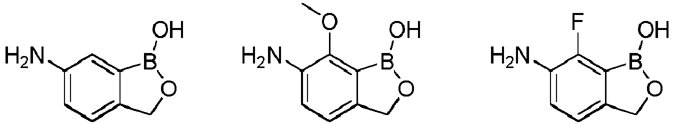 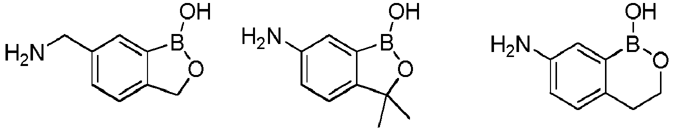 et dans lequel l'hémiester boronate est greffé audit deuxième glycosaminoglycane du fait que le groupe -NH₂ de l'hémiester boronate forme un amide avec un groupe carboxylate de charpente dudit deuxième glycosaminoglycane.

13. Utilisation d'un hémiester boronate dans la fabrication de glycosaminoglycanes réticulés, tels que l'acide hyaluronique réticulé, la réticulation se faisant via un anion ester alcoxyboronate formé entre une fonction diol de charpente d'un premier glycosaminoglycane et un hémiester boronate greffé à un deuxième glycosaminoglycane.

14. Utilisation selon la revendication 13, dans laquelle ledit hémiester boronate est un composé de formule (II) 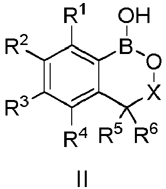 dans laquelle
R¹ est choisi parmi H, F, Cl, NO₂, alkyle en C₁ à C₃, halogénoalkyle en C₁ à C₃ et alcoxy en C₁ à C₃ ;
R², R³ et R⁴ sont indépendamment choisis parmi H, F, Cl, halogénoalkyle en C₁ à C₃, NO₂, alcoxy en C₁ à C₃, alkyle en C₁ à C₃ et un lieur capable de se lier de manière covalente audit deuxième glycosaminoglycane ;
X est choisi parmi CHR⁷ et une liaison ; et
R⁵, R⁶ et R⁷ sont indépendamment choisis parmi H, alkyle en C₁ à C₄, cycloalkyle en C₃ à C₆, phényle, et un cycle hétéroaromatique à cinq à six chaînons comprenant 1 à 3 hétéroatomes choisis parmi O, N et S,
dans laquelle l'un de R², R³ et R⁴ est un lieur, par exemple R² est un lieur.

15. Composition de polymère comprenant des glycosaminoglycanes réticulés selon l'une quelconque des revendications 1 à 6 et un tampon aqueux.
